(19) Europäisches Patentamt
European Patent Office
Office européen des brevets

(11) Veröffentlichungsnummer: **0 214 094 A1**

(12) **EUROPÄISCHE PATENTANMELDUNG**

(21) Anmeldenummer: 86810349.0

(22) Anmeldetag: 08.08.86

(51) Int. Cl.[4]: C 07 D 211/90
C 07 D 409/12, A 61 K 31/44

(30) Priorität: 14.08.85 CH 3504/85

(43) Veröffentlichungstag der Anmeldung: 11.03.87 Patentblatt 87/11

(84) Benannte Vertragsstaaten: AT BE CH DE FR GB IT LI LU NL SE

(71) Anmelder: CIBA-GEIGY AG
Klybeckstrasse 141
CH-4002 Basel(CH)

(72) Erfinder: Eichenberger, Kurt, Dr.
Neubergweg 36
CH-4106 Therwil(CH)

(72) Erfinder: Kühnis, Hans, Dr.
Lange Gasse 26
CH-4052 Basel(CH)

(54) **1,4-Dihydropyridinverbindungen.**

(57) Verbindungen der Formel

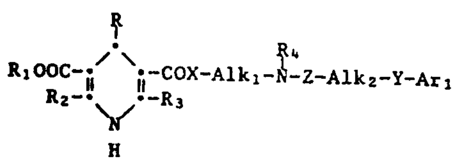

(I),

worin R einen carbocyclischen oder heterocyclischen Arylrest bedeutet, $R_1$ Niederalkyl darstellt, eine der Gruppen $R_2$ und $R_3$ für Niederalkyl und die andere für Niederalkyl, Cyan oder Amino steht, X Sauerstoff oder die Gruppe –NH– darstellt, $Alk_1$ Niederalkylen oder gegebenenfalls substituiertes Phenylniederalkylen bedeutet, das die Gruppe X vom Stickstoffatom durch mindestens zwei Kohlenstoffatome trennt, $R_4$ Wasserstoff, Niederalkyl oder gegebenenfalls substituiertes Phenylniederalkyl bedeutet, Z für die Gruppe –(C=O)– oder eine einfache Bindung steht, $Alk_2$ Niederalkylen darstellt, welches die Gruppen Z und Y vorzugsweise durch 2 - 4 Kohlenstoffatome trennt, Y für die Gruppe –(C=O)– steht, und $Ar_1$ einen monocyclischen carbocyclischen oder heterocyclischen Arylrest darstellt, sowie Salze solcher Verbindungen mit salzbildenden Eigenschaften, zeigen cardiovaskuläre, insbesondere coronardilatatorische und antihypertensive Eigenschaften.

EP 0 214 094 A1

0214094

CIBA-GEIGY AG
Basel (Schweiz)

4-15464/+

BEZEICHNUNG GEÄNDERT
siehe Titelseite

## Ungesättigte Verbindungen

Die Erfindung betrifft neue ungesättigte Verbindungen und deren Salze, Verfahren zu ihrer Herstellung, diese enthaltende pharmazeutische Präparate sowie deren Verwendung.

Die Erfindung betrifft Verbindungen der Formel

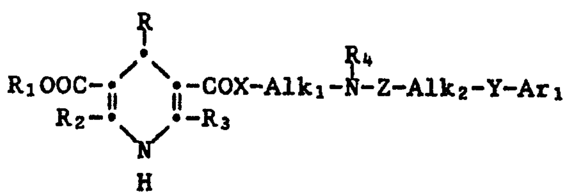
(I),

worin R einen carbocyclischen oder heterocyclischen Arylrest bedeutet, $R_1$ Niederalkyl darstellt, eine der Gruppen $R_2$ und $R_3$ für Niederalkyl und die andere für Niederalkyl, Cyan oder Amino steht, X Sauerstoff oder die Gruppe -NH- darstellt, $Alk_1$ Niederalkylen oder gegebenenfalls substituiertes Phenylniederalkylen bedeutet, das die Gruppe X vom Stickstoffatom durch mindestens zwei Kohlenstoffatome trennt, $R_4$ Wasserstoff, Niederalkyl oder gegebenenfalls substituiertes Phenylniederalkyl bedeutet, Z für die Gruppe -(C=O)- oder eine einfache Bindung steht, $Alk_2$ Niederalkylen darstellt, welches die Gruppen Z und Y vorzugsweise durch 2 - 4 Kohlenstoffatome trennt, Y für die Gruppe -(C=O)- steht, und $Ar_1$ einen monocyclischen carbocyclischen oder heterocyclischen Arylrest darstellt, sowie Salze von Verbindungen der Formel I mit salzbildenden Gruppen.

Ein carbocyclischer oder heterocyclischer Arylrest R ist in erster Linie ein entsprechender monocyclischer Rest, kann jedoch auch ein bi- oder polycyclischer carbocyclischer oder heterocyclischer Rest mit aromatischen Eigenschaften sein.

Carbocyclische Reste R dieser Art sind insbesondere gegebenenfalls substituiertes Phenyl, ferner Naphthyl.

Heterocyclische Arylreste R sind vorzugsweise entsprechende monocyclische Reste, können jedoch auch entsprechende bi- oder polycyclische Reste sein, wobei letztere aus mehreren heterocyclischen Ringen, oder aus einem oder mehreren heterocyclischen Ringen mit einem oder mehreren ankondensierten carbocyclischen Ringen, insbesondere einem oder mehreren ankondensierten Benzoringen bestehen können. Die üblicherweise vorliegenden heterocyclischen Reste R, die vorzugsweise aus fünf oder sechs Ringgliedern bestehen, können bis zu vier, gleiche oder verschiedene Hetero-, insbesondere Stickstoff-, Sauerstoff- und/oder Schwefelatome, vorzugsweise ein, zwei, drei oder vier Stickstoffatome, ein Sauerstoff- oder Schwefelatom, oder ein oder zwei Stickstoffatome zusammen mit einem Sauerstoff- oder Schwefelatom als Ringglieder enthalten. Sie sind mit dem Kohlenstoffatom der 4-Stellung des 1,4-Dihydropyridinringes üblicherweise über ein Ringkohlenstoffatom gebunden.

Monocyclische fünfgliedrige Heteroarylreste R sind z.B. entsprechende monoaza-, diaza-, triaza-, tetraza-, monooxa-, monothia-, oxaza-, oxadiaza-, thiaza- oder thiadiazacyclische Reste, wie Pyrryl-, Pyrazolyl-, Imidazolyl-, Triazolyl-, Tetrazolyl-, Furyl-, Thienyl-, Isoxazolyl-, Oxazolyl-, Oxadiazolyl-, Isothiazolyl-, Thiazolyl- oder Thiadiazolylreste, während monocyclische, sechsgliedrige Heteroarylreste R z.B. entsprechende monoaza-, diaza- oder triazacyclische Reste, wie Pyridyl-, Pyridazinyl-, Pyrimidinyl-, Pyrazinyl- oder Triazinylreste sind. Bicyclische Heteroarylreste sind insbesondere monocyclische Heteroarylreste mit ankondensiertem Benzoring; der Heteroring kann fünf- oder sechsgliedrig sein, wobei

ein fünfgliedriger Heteroarylrest z.B. einen monoaza-, diaza-, diaza-monooxa-, monooxa-, monothia-, oxaza- oder thiazacyclischen Rest, und ein sechsgliedriger Heteroarylrest z.B. einen monoaza- oder einen diazacyclischen Heteroarylrest darstellt. Solche bicyclischen Reste, die über ein Ringkohlenstoffatom des hetero- oder carbocyclischen Restes gebunden sein können, sind z.B. Indolyl-, Isoindolyl-, Benzimidazolyl-, Benzoxadiazolyl-, Benzofuranyl-, Benzofurazanyl-, Benzothienyl-, Benzthiazolyl-, Benzthiadiazolyl-, Chinolinyl- oder Isochinolinylreste, wobei stickstoffhaltige mono- oder bicyclische Heteroarylreste der genannten Art, insbesondere unsubstituierte oder substituierte Pyridylreste, auch als N-Oxide vorliegen können, z.B. Pyridyl-N-oxid.

Ein monocyclischer carbocyclischer Arylrest $Ar_1$ stellt in erster Linie einen unsubstituierten oder ein- oder mehrfach substituierten Phenylrest dar, während ein monocyclischer Heteroarylrest $Ar_1$ beispielsweise einen monocyclischen Monooxa-, Monoaza- oder Monothiaarylrest, etwa solche, wie für R definiert, in erster Linie unsubstituiertes oder ein- oder mehrfach gleich oder verschieden substituiertes Furyl, Pyridyl bzw. Thienyl bedeutet.

Die carbocyclischen und heterocyclischen Arylreste R sowie $Ar_1$ können unsubstituiert oder substituiert sein. Substituenten sind in erster Linie an Ringkohlenstoffatome, aber auch an Ringstickstoffatome gebunden. Sie sind ein- bis dreifach vorhanden und gleich oder verschieden, vorzugsweise jedoch einfach vorhanden. Substituenten von Ringkohlenstoffatomen sind u.a. gegebenenfalls substituierte Kohlenwasserstoffreste, wie entsprechende aliphatische, cycloaliphatische, aromatische oder araliphatische Kohlenwasserstoffreste, z.B. Niederalkyl, Niederalkenyl, Niederalkinyl, Niederalkylen, Cycloalkyl, Cycloalkylniederalkyl, Phenyl oder Phenylniederalkyl. Substituenten von solchen Kohlenwasserstoffresten, insbesondere von Niederalkyl, Phenyl oder Phenylniederalkyl, sind z.B. gegebenenfalls verätherte oder veresterte Hydroxygruppen, wie Hydroxy, gegebenenfalls, z.B. durch gegebenenfalls veräthertes oder verestertes Hydroxy substituiertes Niederalkoxy, z.B. Niederalkoxy,

Niederalkoxyniederalkoxy, oder Halogenniederalkoxy, gegebenenfalls, z.B. durch gegebenenfalls veräthertes oder verestertes Hydroxy, substituiertes Niederalkenyloxy, z.B. Niederalkenyloxy oder Halogenniederalkenyloxy, Niederalkinyloxy, Niederalkylendioxy, Niederalkanoyloxy oder Halogen, und/oder gegebenenfalls funktionell abgewandeltes Carboxy, wie Carboxy, verestertes Carboxy, z.B. Niederalkoxycarbonyl, amidiertes Carboxy, wie Carbamoyl, N-Niederalkyl-carbamoyl oder N,N-Diniederalkyl-carbamoyl, oder Cyan. Cyclische Substituenten, insbesondere Phenyl, können zudem auch Niederalkyl, das gegebenenfalls, z.B. wie angegeben, substituiert sein kann, als Substituenten enthalten. Weitere Substituenten von Arylresten R sowie $Ar_1$ sind z.B. gegebenenfalls verätherte oder veresterte Hydroxygruppen, wie Hydroxy, gegebenenfalls, z.B. durch gegebenenfalls veräthertes oder verestertes Hydroxy substituiertes Niederalkoxy, z.B. Niederalkoxy, Niederalkoxyniederalkoxy oder Halogenniederalkoxy, gegebenenfalls, z.B. durch gegebenenfalls veräthertes oder verestertes Hydroxy substituiertes Niederalkenyloxy, z.B. Niederalkenyloxy oder Halogenniederalkenyloxy, Niederalkinyloxy, Niederalkylendioxy, Niederalkanoyloxy oder Halogen, Nitro, gegebenenfalls substituiertes Amino, wie Amino, N-Niederalkylamino, N,N-Diniederalkylamino, N-Niederalkyl-N-phenylniederalkylamino, Niederalkylenamino, Oxaniederalkylenamino, Thianiederalkylenamino oder Azaniederalkylenamino, wobei der Aza-Stickstoff unsubstituiert oder, z.B. durch gegebenenfalls, z.B. wie oben beschrieben, substituiertes Niederalkyl, Phenyl oder Phenylniederalkyl substituiert sein kann, oder Acylamino, z.B. Niederalkanoylamino, Azido, Acyl, wie Niederalkanoyl oder gegebenenfalls funktionell abgewandeltes Carboxy, wie Carboxy, verestertes Carboxy, z.B. Niederalkoxycarbonyl, oder amidiertes Carboxy, wie Carbamoyl, N-Niederalkyl-carbamoyl oder N,N-Diniederalkyl-carbamoyl, ferner Cyan, gegebenenfalls funktionell abgewandeltes Sulfo, wie Sulfo, Aminosulfonyl, N-Niederalkylaminosulfonyl oder N,N-Diniederalkylaminosulfonyl und/oder veräthertes Mercapto, das gegebenenfalls oxidiert sein kann, wie Niederalkylthio, Niederalkylsulfinyl oder

Niederalkylsulfonyl. Substituenten von Ringstickstoffatomen sind in erster Linie die obengenannten, gegebenenfalls substituierten Kohlenwasserstoffreste, wie Niederalkyl, ferner Hydroxy oder Oxido.

Heterocyclische Arylreste R bzw. $Ar_1$ können, z.B. je nach Art der Substitution, in verschiedenartigen tautomeren Formen vorliegen.

Die vor- sowie nachstehend verwendeten Definitionen haben, sofern nicht besonders definiert, die folgenden Bedeutungen:

Der Ausdruck "nieder" bedeutet, dass entsprechend definierte Gruppen oder Verbindungen, sofern nicht anders definiert, bis und mit 7, vorzugsweise bis und mit 4, Kohlenstoffatome enthalten.

Substituierte Reste können einen oder mehrere, gleiche oder verschiedene Substituenten enthalten; diese können irgendeine geeignete Stellung einnehmen.

Naphthyl kann 1- oder 2-Naphthyl sein.

Pyrryl ist z.B. 2- oder 3-Pyrryl, Pyrazolyl z.B. 3- oder 4-Pyrazolyl, Imidazolyl z.B. 2- oder 4-Imidazolyl, Triazolyl z.B. 1,3,5-1H-Triazol-2-yl oder 1,3,4-Triazol-2-yl, und Tetrazolyl z.B. 1,2,3,4-1H-Tetrazol-5-yl, während Furyl 2- oder 3-Furyl und Thienyl 2- oder 3-Thienyl bedeuten. Isoxazolyl ist z.B. 3-Isoxazolyl, Oxazolyl z.B. 2- oder 4-Oxazolyl, Oxadiazolyl z.B. 1,3,4-Oxadiazol-2-yl, Thiazolyl z.B. 4-Thiazolyl und Thiadiazolyl z.B. 1,3,4-Thiadiazolyl-2-yl.

Pyridyl ist 2-, 3- oder 4-Pyridyl, Pyridazinyl z.B. 3-Pyridazinyl, Pyrimidinyl 2-, 4- oder 5-Pyrimidinyl, Pyrazinyl, 2-Pyrazinyl und Triazinyl z.B. 1,3,5-Triazin-2-yl.

Indolyl ist z.B. 2-, 3- oder 5-Indolyl, Isoindolyl z.B. 1-Isoindolyl, Benzimidazolyl z.B. 2- oder 5-Benzimidazolyl, Benzofuranyl z.B. 2- oder 3-Benzofuranyl, Benzofurazanyl (auch 2,1,3-Benzoxadiazolyl) z.B. 4-Benzofurazanyl, Benzothienyl z.B. 3-Benzothienyl, Benzthiazolyl z.B. 2-Benzthiazolyl, 2,1,3-Benzthiadiazolyl z.B. 2,1,3-Benzthiadiazol-4-yl, Chinolinyl z.B. 2- oder 4-Chinolinyl, und Isochinolinyl z.B. 1-Isochinolinyl.

Niederalkyl ist z.B. Methyl, Aethyl, n-Propyl, Isopropyl, n-Butyl, Isobutyl oder tert.-Butyl, ferner n-Pentyl, n-Hexyl oder n-Heptyl, während Niederalkenyl z.B. Allyl oder Methallyl, und Niederalkinyl z.B. Propargyl bedeutet.

Cycloalkyl weist vorzugsweise 5 bis 7 Ringkohlenstoffatome auf und ist z.B. Cyclopentyl oder Cyclohexyl, während Cycloalkylniederalkyl z.B. Cyclopropylmethyl, Cyclopentylmethyl oder Cyclohexylmethyl sein kann.

Phenylniederalkyl ist z.B. Benzyl oder 1- oder 2-Phenyläthyl, wobei Substituenten, insbesondere des Phenylteils, gegebenenfalls verätherte oder veresterte Hydroxygruppen, wie Hydroxy oder Halogen, gegebenenfalls, z.B. durch gegebenenfalls veräthertes oder verestertes Hydroxy substituiertes Niederalkyl oder Niederalkoxy, z.B. Niederalkyl, Hydroxyniederalkyl, Halogenniederalkyl, Niederalkoxy, Niederalkoxyniederalkoxy oder Halogenniederalkoxy sind.

Niederalkoxy steht in erster Linie für Methoxy, Aethoxy, n-Propyloxy, Isopropyloxy, n-Butyloxy, Isobutyloxy oder tert.-Butyloxy.

In einem Niederalkoxyniederalkoxyrest ist die terminale Niederalkoxygruppe vorzugsweise durch mehr als ein Kohlenstoffatom vom Verknüpfungssauerstoffatom getrennt; solche Reste sind z.B. 2-Methoxyäthoxy oder 2-Aethoxy-äthoxy.

In einem Halogen-niederalkoxyrest können ein oder mehrere Halogenatome, die vorzugsweise eine Atomnummer bis 35 aufweisen und besonders für Fluor und/oder Chlor stehen, vorhanden sein; solche Reste sind z.B. Difluormethoxy oder 1,1,2-Trifluor-2-chlor-äthoxy.

Niederalkenyloxy ist z.B. Allyloxy oder Methallyloxy, und Halogenniederalkenyloxy, das ein oder mehrere Halogenatome enthalten kann, wobei letztere vorzugsweise eine Atomnummer bis 35 aufweisen und besonders für Fluor und/oder Chlor stehen, ist z.B. 1,2-Dichlorvinyloxy.

Niederalkinyloxy ist z.B. Propargyloxy, während Niederalkylendioxy z.B. Methylendioxy oder Aethylendioxy bedeutet.

Niederalkylen $Alk_1$ und $Alk_2$ haben jeweils bis zu 8 Kohlenstoffatome, wobei $Alk_1$ die Gruppe X und das Stickstoffatom durch 2 bis 8, vorzugsweise durch 2 bis 4 Kohlenstoffatome trennt. Niederalkylen $Alk_2$ trennt die Gruppen Z und Y durch 2 bis 6, vorzugsweise durch 2 bis 4 Kohlenstoffatome. Beide Reste $Alk_1$ und $Alk_2$ sind jeweils z.B. Aethylen, 1,2- oder 1,3-Propylen, 1,4-Butylen, 1,5-Pentylen, 1,6-Hexylen, 1,7-Heptylen oder 1,8-Octylen.

Gegebenenfalls substituiertes Phenylniederalkylen $Alk_1$ hat im Alkylenteil ebenfalls bis zu 8 Kohlenstoffatome und trennt die Gruppe X und das Stickstoffatom durch 2 bis 8, vorzugsweise durch 2 bis 4 Kohlenstoffatome. Diese Reste $Alk_1$ werden z.B. durch 1-Phenyl-1,2-äthylen, 2-Phenyläthylen oder 1- oder 2-Phenyl-1,3-propylen repräsentiert.

Niederalkylendioxy ist z.B. Methylendioxy oder 1,2-Aethylendioxy.

Niederalkanoyloxy ist z.B. Acetyloxy, Propionyloxy oder Pivaloyloxy.

Halogen hat vorzugsweise eine Atomnummer bis und mit 35 und ist insbesondere Fluor oder Chlor, ferner Brom, kann jedoch auch Iod sein.

Hydroxysubstituiertes Niederalkyl ist z.B. 2-Hydroxyäthyl.

Halogensubstituiertes Niederalkyl ist z.B. Trifluormethyl, 1,1,2-Trifluor-2-chlor-äthyl oder Chlormethyl.

Niederalkoxycarbonyl ist z.B. Methoxycarbonyl, Aethoxycarbonyl, n-Propyloxycarbonyl, Isopropyloxycarbonyl, n-Butyloxycarbonyl, Isobutyloxycarbonyl oder tert.-Butyloxycarbonyl.

N-Niederalkyl-carbamoyl ist z.B. N-Methyl-carbamoyl oder N-Aethylcarbamoyl, während N,N-Diniederalkyl-carbamoyl z.B. N,N-Dimethyl-carbamoyl oder N,N-Diäthyl-carbamoyl ist.

N-Niederalkylamino ist z.B. N-Methylamino, N-Aethylamino, N-n-Propylamino oder N-Isopropylamino.

N,N-Diniederalkylamino ist z.B. N,N-Dimethylamino, N-Aethyl-N-methylamino oder N,N-Diäthylamino, während N-Niederalkyl-N-phenylniederalkylamino z.B. N-Benzyl-N-methylamino oder N-methyl-N-(2-phenyläthyl)-amino darstellt.

Niederalkylenamino enthält vorzugsweise 4 bis 6 Ringkohlenstoffatome und ist z.B. Pyrrolidino oder Piperidino, während Oxaniederalkylenamino z.B. 4-Morpholino, Thianiederalkylenamino z.B. 4-Thiomorpholino, und gegebenenfalls azasubstituiertes Azaniederalkylenamino z.B. Piperazino, 4-Methylpiperazino, 4-Phenyl-piperazino, 4-Benzylpiperazino oder 4-(2-Phenyläthyl)-piperazino darstellen kann.

Niederalkanoylamino ist z.B. Acetylamino oder Propionylamino.

Niederalkanoyl ist z.B. Formyl, Acetyl, Propionyl oder Pivaloyl.

Niederalkylthio ist z.B. Methylthio, Aethylthio, n-Propylthio oder Isopropylthio, während Niederalkylsulfinyl z.B. Methylsulfinyl, und Niederalkylsulfonyl z.B. Methylsulfonyl oder Aethylsulfonyl bedeuten.

N-Niederalkylaminosulfonyl ist z.B. N-Methylaminosulfonyl, während N,N-Diniederalkylaminosulfonyl z.B. N,N-Dimethylaminosulfonyl ist.

In einem substituierten Niederalkoxycarbonyl ist der Substituent üblicherweise durch mindestens 2, vorzugsweise durch 2 oder 3 Kohlenstoffatome vom Sauerstoff getrennt. Solche Reste sind z.B. Hydroxyniederalkoxycarbonyl, wie 2-Hydroxyäthoxycarbonyl oder 2,3-Dihydroxypropyloxycarbonyl, Niederalkoxy-niederalkoxycarbonyl, z.B. 2-Methoxyäthoxycarbonyl, Diniederalkylamino-niederalkoxycarbonyl, z.B. 2-Dimethylaminoäthoxycarbonyl, 2-Diäthylaminoäthoxycarbonyl oder 3-Dimethylaminopropyloxycarbonyl, Niederalkylenaminoniederalkoxycarbonyl, z.B. 2-Pyrrolidinoäthoxycarbonyl oder 2-Piperidinoäthoxycarbonyl, Morpholino-niederalkoxycarbonyl, z.B. 2-(4-Morpholino)-äthoxycarbonyl, oder (4-Niederalkyl-piperazino)-niederalkoxycarbonyl, z.B. 2-(4-Methylpiperazino)-äthoxycarbonyl.

Phenylniederalkoxycarbonyl ist z.B. Benzyloxycarbonyl oder 2-Phenyläthoxy-carbonyl.

N,N-Niederalkylen-carbamoyl ist z.B. Pyrrolidinocarbonyl oder Piperidinocarbonyl, wobei entsprechende Reste, in welchen der Niederalkylenteil durch Sauerstoff, Schwefel oder unsubstituierten oder substituierten Stickstoff unterbrochen ist, z.B. 4-Morpholinocarbonyl, 4-Thiomorpholinocarbonyl, 1-Piperazinocarbonyl oder 4-Methyl-1-piperazinocarbonyl bedeuten.

Verbindungen der Formel I mit salzbildenden basischen Eigenschaften können in Form von Salzen, insbesondere von Säureadditionssalzen, in erster Linie entsprechenden pharmazeutisch verwendbaren, nicht-toxischen Säureadditionssalzen, vorliegen. Solche Salze sind z.B. diejenigen mit Mineralsäuren, wie Halogenwasserstoffsäuren, z.B. Chlorwasserstoffsäure oder Bromwasserstoffsäure, ferner Salpetersäure, Schwefelsäure oder Phosphorsäure, oder organischen Säuren, wie Carbonsäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Amino-salicylsäure, 2-Phenoxybenzoesäure, 2-Acetoxybenzoesäure, Embonsäure, Nicotinsäure oder Isonicotinsäure, ferner Aminosäuren, oder organischen Sulfonsäuren,

wie gegebenenfalls Hydroxy enthaltende Niederalkansulfonsäuren, z.B. Methansulfonsäure, Aethansulfonsäure, 2-Hydroxyäthansulfonsäure oder Aethan-1,2-disulfonsäure, oder Arylsulfonsäuren, z.B. Benzolsulfonsäure, 4-Methyl-benzolsulfonsäure oder Naphthalin-2-sulfonsäure, oder mit anderen sauren organischen Substanzen, wie Ascorbinsäure.

Salze von Ausgangsstoffen mit salzbildenden basischen Eigenschaften sind z.B. solche mit Mineralsäuren, etwa Salzsäure, Schwefelsäure oder Phosphorsäure, oder mit organischen Säuren, z.B. Essigsäure.

Die Verbindungen der Formel I und deren Salze besitzen wertvolle pharmakologische Eigenschaften, vor allem im cardiovasculären Bereich. Sie wirken als Calcium-Antagonisten und besitzen α-Rezeptoren blockierende sowie Serotonin-antagonistische Eigenschaften, wie sich z.B. in in-vitro-Versuchen an isoliert perfundierten Mesenterialgefässen der Ratte [Mc Gregor, D.: J.Physiol. 177, 21 - 30, (1965)] anhand der Hemmung der Kalium- und Noradrenalininduzierten Vasokonstriktion, beispielsweise unter Verwendung von 2,6-Dimethyl-4-(3-nitrophenyl)-1,4-dihydropyridin-3,5-dicarbonsäure-3-methylester-5-[2-[N-(4-(4-fluorphenyl)-4-oxo-n-butyl)-N-methyl-amino]äthylester] bei einer Konzentration von ca. $10^{-8}$ Mol/Liter ($IC_{50}$) für die Hemmung der Kalium-induzierten Vasokonstriktion, und von ca. $3 \cdot 10^{-10}$ Mol/Liter ($IC_{50}$) für die Hemmung der Noradrenalininduzierten Vasokonstriktion zeigen lässt. Ferner lässt sich die Bindung der neuen Verbindungen oder deren Salze an $Serotonin_2$-Rezeptoren mittels Verdrängung von $^3H$-Ketanserin aus seinen Bindungsstellen an Membranen des Rattengehirns [Leysen J.E. et al.: Molecul. Pharmacol. 21, 301 - 304 (1982)] z.B. unter Verwendung von 2,6-Dimethyl-4-(3-nitrophenyl)-1,4-dihydropyridin-3,5-dicarbonsäure-3-methylester-5-[2-[N-(4-(4-fluorphenyl)-4-oxo-n-butyl)-N-methyl-amino]äthylester] bei einer Konzentration von ca. $10^{-8}$ Mol/Liter ($IC_{50}$) nachweisen.

Die neuen Verbindungen oder deren Salze wirken ausserdem antihypertensiv, wie sich z.B. durch Senkung des Blutdrucks an der renal hypertonischen Ratte zeigen lässt. So kann z.B. 2 Stunden nach p.o.-Applikation von 15 mg/kg 2,6-Dimethyl-4-(3-nitrophenyl)-1,4-dihydropyridin-3,5-dicarbonsäure-3-methylester-5-[2-[N-(4-(4-fluorphenyl)-4-oxo-n-butyl)-N-methyl-amino]äthylester] an der renal hypertonischen Ratte eine Blutdrucksenkung von ca. 45 mm Hg beobachtet werden.

Die Verbindungen der Formel I und Salze von solchen Verbindungen können daher z.B. als Coronardilatatoren und Antihypertensiva zur Behandlung von cardiovasculären Krankheitszuständen, wie Angina pectoris und ihre Folgen, Gefäss-Spasmen, zentrale und periphere Durchblutungsstörungen, hoher Blutdruck, Arrhythmien und Herzinsuffizienz, ferner zur Hemmung der Plättchenaggregation oder zur Behandlung von Migräne Verwendung finden. Die neuen Verbindungen sind aber auch wertvolle Zwischenprodukte zur Herstellung anderer, insbesondere pharmazeutisch wirksamer Verbindungen.

Die Erfindung betrifft insbesondere neue Verbindungen der Formel I, in welchen R für einen mono- oder bicyclischen carbocyclischen Arylrest oder für einen fünf- oder sechsgliedrigen, ein bis vier Ringstickstoffatome, ein Ringsauerstoff- oder Ringschwefelatom, oder ein oder zwei Ringstickstoffatome zusammen mit einem Ringsauerstoff- oder einem Ringschwefelatom als Ringglieder enthaltenden, monocyclischen Heteroarylrest steht, der über ein Ringkohlenstoffatom mit dem Kohlenstoffatom der 4-Stellung des 1,4-Dihydropyridinrings verbunden ist, und der gegebenenfalls einen ankondensierten Benzoring enthält, und insbesondere Phenyl, Naphthyl, Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl, Triazinyl, Indolyl, Isoindolyl, Benzimidazolyl, Benzoxadiazolyl, Benzofuranyl, Benzofurazanyl, Benzothienyl, Benzthiazolyl, 2,1,3-Benzthiadiazolyl, Chinolinyl oder Isochinolinyl bedeutet, wobei in diesen Resten Ringkohlenstoffatome gegebenenfalls durch Niederalkyl, Niederalkenyl, Niederalkinyl, Niederalkylen, Cycloalkyl, Phenyl und/oder Phenylniederalkyl, wobei Niederalkyl, Phenyl oder Phenylniederalkyl gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, und cyclische Reste auch Niederalkyl, das seinerseits wie angegeben substituiert sein kann, als Substituenten enthalten können, und/oder durch Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Nitro, Amino, N-Niederalkyl-amino, N,N-Diniederalkylamino, N-Niederalkyl-N-phenylniederalkyl-amino, Niederalkylenamino, Oxaniederalkylenamino, Thianiederalkylenamino und/oder Azaniederalkylenamino, worin das Azastickstoffatom durch Niederalkyl, Phenyl oder Phenylniederalkyl substituiert sein kann, wobei diese Reste Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, die cyclischen Reste auch Niederalkyl als Substituenten enthalten können, und/oder durch Niederalkanoylamino, Azido, Niederalkanoyl, Carboxy, Niederalkoxycarbonyl, Carbamoyl, N-Niederalkyl-carbamoyl, N,N-Diniederalkyl-carbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl, und/oder Ringstickstoffatome gegebenenfalls durch Niederalkyl, das als Substituenten gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl oder Cyan enthalten kann, oder durch Hydroxy oder Oxido substituiert sein können, $R_1$ Niederalkyl ist, einer der Reste $R_2$ und $R_3$ für Niederalkyl und der andere für Niederalkyl, Cyan oder Amino steht, X Sauerstoff oder die

Gruppe -NH- bedeutet, $Alk_1$ Niederalkylen oder gegebenenfalls insbesondere im Phenylteil durch gegebenenfalls veräthertes oder verestertes Hydroxy, durch gegebenenfalls veräthertes oder verestertes Hydroxy substituiertes Niederalkyl oder Niederalkoxy oder durch Carboxy, Niederalkoxycarbonyl oder Cyan substituiertes Phenylniederalkylen darstellt, das die Gruppe X vom Stickstoffatom durch 2 bis 8 Kohlenstoffatome trennt, $R_4$ Wasserstoff, Niederalkyl, gegebenenfalls insbesondere im Phenylteil durch gegebenenfalls veräthertes oder verestertes Hydroxy, durch gegebenenfalls veräthertes oder verestertes Hydroxy substituiertes Niederalkyl oder Niederalkoxy substituiertes Phenylniederalkyl bedeutet, Z die Gruppe -(C=O)- oder eine einfache Bindung darstellt, $Alk_2$ Niederalkylen bedeutet, welches die Gruppen Z und Y durch 2 bis 6 Kohlenstoffatome trennt, Y für die Gruppe -(C=O)- steht, und $Ar_1$ einen monocyclischen carbocyclischen Aryl- oder Heteroarylrest darstellt, und insbesondere Phenyl, Naphthyl, Pyrryl, Furyl, Thienyl oder Pyridyl bedeutet, wobei diese Reste wie vorstehend im Zusammenhang mit R definiert substituiert sein können, sowie Salze insbesondere pharmazeutisch verwendbare, nichttoxische Säureadditionssalze solcher Verbindungen mit salzbildenden basischen Eigenschaften.

Die Erfindung betrifft in erster Linie neue Verbindungen der Formel I, worin R und $Ar_1$ jeweils für Phenyl oder Naphthyl stehen, das gegebenenfalls durch Niederalkyl, Phenyl und/oder Phenylniederalkyl, wobei solche Reste ihrerseits Hydroxy, Niederalkoxy, Halogenniederalkoxy, Niederalkylendioxy, Halogen, Carboxy, Niederalkoxycarbonyl und/oder Cyan, die cyclischen Reste auch Niederalkyl als Substituenten enthalten können, und/oder durch Hydroxy, Niederalkoxy, Halogenniederalkoxy, Niederalkenyloxy, Halogenniederalkenyloxy, Niederalkylendioxy, Halogen, Nitro, Amino, N-Niederalkylamino, N,N-Diniederalkylamino, Niederalkanoylamino, Carboxy, Niederalkoxycarbonyl, Carbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl substituiert ist, oder R und $Ar_1$ jeweils für über ein Ringkohlenstoffatom gebundenes Pyrryl, Furyl, Thienyl, Pyridyl, 1-Oxido-pyridyl oder Imidazolyl,

R auch für Benzofurazanyl oder Benzoxadiazolyl steht, wobei solche Reste gegebenenfalls, wie für einen Phenyl- oder Naphthylrest R bzw. $Ar_1$ angegeben, substituiert sind, und insbesondere Niederalkyl, Niederalkoxy, Halogen und/oder gegebenenfalls durch Niederalkyl, Niederalkoxy, Halogen und/oder Nitro substituiertes Phenyl als Substituenten enthalten können, $R_1$ Niederalkyl ist, $R_2$ und $R_3$ jeweils Niederalkyl bedeuten, oder eine der Gruppen $R_2$ und $R_3$ Niederalkyl ist und die andere Niederalkyl oder Cyan darstellt, X Sauerstoff oder die Gruppe -NH- bedeutet, $Alk_1$ für die Gruppe $-(CH_2)_n-$ steht, worin n Werte von 2 bis 6 darstellt, $R_4$ Wasserstoff, Niederalkyl oder Phenylniederalkyl bedeutet, Z die Gruppe -(C=O)- oder eine einfache Bindung darstellt, $Alk_2$ für die Gruppe $-(CH_2)_m-$ steht, worin m Werte von 2 bis 6, vorzugsweise 2 bis 4 bedeutet, und Y für die Gruppe -(C=O)- steht, sowie Salze, insbesondere pharmazeutisch verwendbare, nichttoxische Säureadditionssalze solcher Verbindungen mit salzbildenden basischen Eigenschaften. Die Erfindung betrifft zusätzlich Verbindungen der Formel I wie unmittelbar vorstehend definiert, in denen $Alk_1$ für die Gruppe $-(CH_2)_n-$ steht, worin n Werte von 2 bis 6 darstellt und worin ein Wasserstoff durch Phenyl ersetzt ist, das gegebenenfalls durch Niederalkyl, Hydroxy, Niederalkoxy, Halogenniederalkoxy, Niederalkylendioxy, Halogen, Carboxy, Niederalkoxycarbonyl und/oder Cyan substituiert ist.

Die Erfindung betrifft ganz besonders neue Verbindungen der Formel I, worin R und $Ar_1$ jeweils für Phenyl stehen, das gegebenenfalls durch Niederalkyl, Hydroxy, Niederalkoxy, Halogenniederalkoxy, Halogenniederalkenyloxy, Niederalkylendioxy, Halogen, Trifluormethyl, Nitro, Niederalkanoylamino, gegebenenfalls durch gegebenenfalls verestertes oder veräthertes Hydroxy substituiertes Phenyl oder Phenylniederalkyl, und/oder Cyan substituiert ist, wobei ein Phenylrest R bzw. $Ar_1$ einen oder mehrere, gleiche oder verschiedene der genannten Substituenten aufweisen kann, oder R und $Ar_1$ jeweils Pyridyl, Furyl, 1-Oxido-pyridyl oder Thienyl, R auch Benzofurazanyl bedeuten, das durch Niederalkyl oder Halogen substituiert sein kann, $R_1$, $R_2$ und $R_3$ jeweils Niederalkyl bedeuten, X für Sauerstoff oder

die Gruppe -NH- steht, $Alk_1$ und $Alk_2$ jeweils die Gruppe $-(CH_2)_n-$ darstellen, worin n Werte von 2 bis 4 bedeutet, $R_4$ Wasserstoff oder Niederalkyl bedeutet, Z die Gruppe -(C=O)- oder eine einfache Bindung darstellt, und Y für die Gruppe -(C=O)- steht, sowie Salze, insbesondere pharmazeutisch verwendbare nicht-toxische Säureadditionssalze solcher Verbindungen mit salzbildenden basischen Eigenschaften. Die Erfindung betrifft zusätzlich Verbindungen der Formel I wie unmittelbar vorstehend definiert, in denen $Alk_1$ die Gruppe $-(CH_2)_n-$ darstellt, worin n Werte von 2 bis 4 bedeutet und worin ein Wasserstoff durch Phenyl ersetzt ist, das gegebenenfalls durch Niederalkyl, Hydroxy, Niederalkoxy, Halogenniederalkoxy, Niederalkylendioxy, Halogen, Carboxy, Niederalkoxycarbonyl und/oder Cyan substituiert ist.

Die Erfindung betrifft insbesondere neue Verbindungen der Formel I, worin R für unsubstituiertes oder vorzugsweise durch Niederalkyl, Niederalkoxy, Halogenniederalkoxy oder Halogenniederalkenyloxy, worin Halogen eine Atomnummer bis und mit 35 hat, Halogen mit einer Atomnummer bis und mit 35, Trifluormethyl, Nitro und/oder Cyan mono- oder di-substituiertes Phenyl steht, wobei Substituenten die 2-und/oder 3-Stellung einnehmen, $R_1$, $R_2$ und $R_3$ jeweils Niederalkyl bedeuten, X für Sauerstoff oder die Gruppe -NH- steht, $Alk_1$ und $Alk_2$ jeweils die Gruppe $-(CH_2)_n-$ darstellen, worin n Werte von 2 bis 4 bedeutet, $R_4$ Wasserstoff oder Niederalkyl darstellt, Z für eine einfache Bindung steht, Y für die Gruppe -(C=O)- steht, und $Ar_1$ gegebenenfalls durch Halogen mit einer Atomnummer bis und mit 35 substituiertes Phenyl oder Pyridyl, z.B. 2-Pyridyl, 3-Pyridyl oder 4-Pyridyl bedeutet, sowie Salze, insbesondere pharmazeutisch verwendbare, nicht-toxische Säureadditionssalze solcher Verbindungen. Die Erfindung betrifft zusätzlich Verbindungen der Formel I wie unmittelbar vorstehend definiert, in denen $Alk_1$ die Gruppe $-(CH_2)_n-$ darstellt, worin n Werte von 2 bis 4 bedeutet und worin ein Wasserstoff durch Phenyl ersetzt ist, das gegebenenfalls durch Niederalkyl, Niederalkoxy, Hydroxy oder Halogen substituiert ist.

Die Erfindung betrifft insbesondere neue Verbindungen der Formel I, worin R für durch Halogen mit einer Atomnummer bis und mit 35, z.B. Fluor, Chlor oder Brom, mono- oder disubstituiertes oder durch Trifluormethyl, Nitro oder Cyan monosubstituiertes Phenyl steht, wobei Substituenten die 2- und/oder 3-Stellung einnehmen, $R_1$, $R_2$ und $R_3$ jeweils Niederalkyl, in erster Linie Methyl, bedeuten, X für Sauerstoff oder die Gruppe -NH- steht, $Alk_1$ und $Alk_2$ jeweils die Gruppe $-(CH_2)_n-$ darstellen, worin n Werte von 2 bis 3 bedeutet, $R_4$ Wasserstoff oder Niederalkyl, in erster Linie Methyl, darstellt, Z eine einfache Bindung ist, Y für die Gruppe -(C=O)- steht, und $Ar_1$ gegebenenfalls durch Halogen mit einer Atomnummer bis und mit 35, z.B. Fluor, substituiertes Phenyl ist, sowie Salze, insbesondere pharmazeutisch verwendbare, nicht-toxische Säureadditionssalze solcher Verbindungen. Die Erfindung betrifft zusätzlich Verbindungen der Formel I wie unmittelbar vorstehend definiert, in denen $Alk_1$ die Gruppe $-(CH_2)_n-$ darstellt, worin n Werte von 2 bis 3 bedeutet und worin ein Wasserstoff durch Phenyl ersetzt ist.

Die Erfindung betrifft insbesondere neue Verbindungen der Formel I, worin R für durch Halogen mit einer Atomnummer von bis und mit 35, z.B. Fluor, Chlor oder Brom, mono- oder disubstituiertes oder durch Nitro oder Cyan monosubstituiertes Phenyl steht, wobei Substituenten die 2- und/oder 3-Stellung einnehmen, $R_1$, $R_2$ und $R_3$ jeweils Niederalkyl, in erster Linie Methyl bedeuten, X für Sauerstoff steht, $Alk_1$ und $Alk_2$ jeweils die Gruppe $-(CH_2)_n-$ darstellen, worin n die Werte von 2 bis 3 bedeutet, $R_4$ Niederalkyl, in erster Linie Methyl darstellt, Z eine einfache Bindung ist, Y für die Gruppe -(C=O)- steht und $Ar_1$ durch Halogen mit einer Atomnummer bis und mit 35, z.B. Fluor, substituiertes Phenyl ist, sowie Salze, insbesondere pharmazeutisch verwendbare, nicht-toxische Säureadditionssalze solcher Verbindungen.

Die Erfindung betrifft insbesondere Verbindungen der Formel I, worin R für in 2- und/oder 3-Stellung durch Halogen mit einer Atomnummer von bis und mit 35 mono- oder disubstituiertes oder durch Nitro oder Cyan monosubstituiertes Phenyl steht, $R_1$, $R_2$ und $R_3$ Niederalkyl mit

bis zu 4 Kohlenstoffatomen bedeuten, X für Sauerstoff steht, $Alk_1$ für unsubstituiertes oder durch Phenyl substituiertes α,ω-Alkylen mit 2 oder 3 Kohlenstoffatomen steht, $Alk_2$ für α,ω-Alkylen mit 2 oder 3 Kohlenstoffatomen steht, $R_4$ für unsubstituiertes oder durch Phenyl substituiertes Niederalkyl mit bis zu 4 Kohlenstoffatomen steht, Z eine einfache Bindung ist, Y für die Gruppe -(C=O)- steht und $Ar_1$ unsubstituiertes oder durch Halogen mit einer Atomnummer von bis und mit 35 substituiertes Phenyl oder Thienyl ist, sowie Salze, insbesondere pharmazeutisch verwendbare, nicht-toxische Säureadditionssalze solcher Verbindungen.

Die Erfindung betrifft insbesondere Verbindungen der Formel I, worin R für in 2- und/oder 3-Stellung durch Halogen mit einer Atomnummer von bis und mit 35 mono- oder disubstituiertes oder durch Nitro oder Cyan monosubstituiertes Phenyl steht, $R_1$, $R_2$ und $R_3$ Niederalkyl mit bis zu 4 Kohlenstoffatomen bedeuten, X für Sauerstoff steht, $Alk_1$ und $Alk_2$ für unsubstituiertes α,ω-Alkylen mit 2 oder 3 Kohlenstoffatomen stehen, $R_4$ für Niederalkyl mit bis zu 4 Kohlenstoffatomen steht, Z eine einfache Bindung ist, Y für die Gruppe -(C=O)- steht und $Ar_1$ durch Halogen mit einer Atomnummer von bis und mit 35 monosubstituiertes Phenyl ist, sowie Salze, insbesondere pharmazeutisch verwendbare, nicht-toxische Säureadditionssalze solcher Verbindungen.

Die Erfindung betrifft insbesondere die in den Beispielen beschriebenen spezifischen Verbindungen.

Die Verbindungen der Formel I und Salze von solchen Verbindungen mit salzbildenden Eigenschaften können in an sich bekannter Weise hergestellt werden, indem man z.B.

a) eine Verbindung der Formel

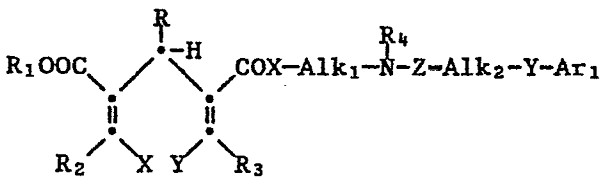

(II),

worin einer der Reste X und Y für die Gruppe der Formel $-NH_2$ steht und der andere Hydroxy oder die Gruppe der Formel $-NH_2$ bedeutet, oder ein Tautomeres davon oder ein entsprechendes Tautomerengemisch ringschliesst, oder

b) eine Verbindung der Formel R-CHO (III) oder ein reaktionsfähiges funktionelles Derivat davon mit einer Verbindung der Formel

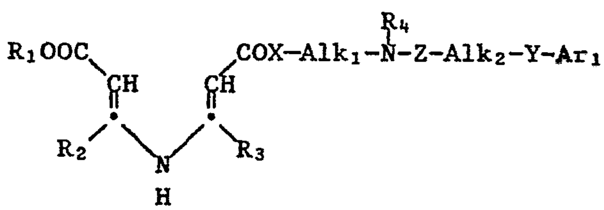

(IV)

oder einem Tautomeren davon oder einem entsprechenden Tautomerengemisch umsetzt, oder

c) in einer Verbindung der Formel

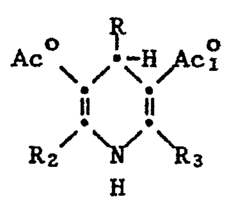

(V),

in welcher einer der Reste $Ac^o$ und $Ac_1^o$ eine in die Gruppe $-COOR_1$ bzw. in den Rest der Formel

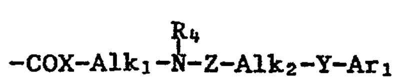

(Va)

überführbare Gruppe bedeutet, und der andere die Gruppe $-COOR_1$ oder die Gruppe der Formel Va oder einen in die Gruppe $-COOR_1$ bzw. die Gruppe der Formel Va überführbaren Rest bedeutet, den Rest $Ac^o$ in die Gruppe $-COOR_1$ und/oder den Rest $Ac_1^o$ in einen Rest der Formel Va überführt, oder

d) eine Verbindung der Formel

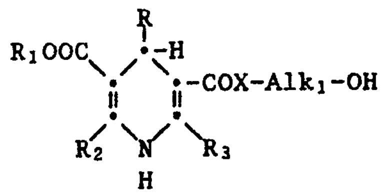

(VI)

oder einen reaktionsfähigen Ester davon, mit einer Verbindung der Formel

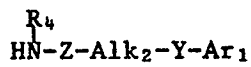

(VIa)

umsetzt, oder

e) eine Verbindung der Formel

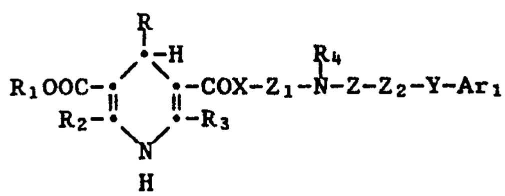

(VII)

worin mindestens eine der Gruppen $Z_1$ und $Z_2$ eine mittels Reduktion in die Gruppe $Alk_1$ bzw. $Alk_2$ überführbare Gruppe bedeutet und die andere die Gruppe $Alk_1$ bzw. $Alk_2$ oder eine mittels Reduktion in die Gruppe $Alk_1$ bzw. $Alk_2$ überführbare Gruppe darstellt, die Gruppe $Z_1$ und/oder die Gruppe $Z_2$ mittels Reduktion in die Gruppe $Alk_1$ bzw. $Alk_2$ überführt, oder

f) eine Verbindung der Formel

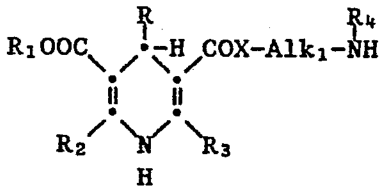

(VIII),

oder ein Salz davon, mit einer Verbindung der Formel

$$HO-Z-Alk_2-Y-Ar_1 \qquad (IX),$$

oder einem reaktionsfähigen Derivat davon, umsetzt, oder

g) in einer Verbindung der Formel

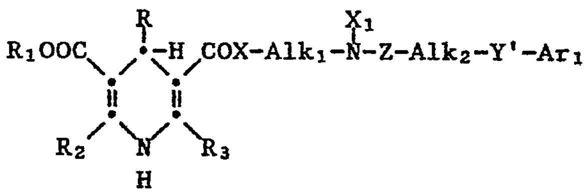

(X),

worin $X_1$ die Bedeutung von $R_4$ hat oder eine abspaltbare und durch Wasserstoff ersetzbare Gruppe bedeutet, Y' die Bedeutung von Y hat oder eine in die Gruppe Y überführbare Gruppe darstellt, wobei mindestens eine abspaltbare und durch Wasserstoff ersetzbare Gruppe $X_1$ abgespalten und durch Wasserstoff ersetzt und/oder eine von Y verschiedene Gruppe Y' in die Gruppe Y umgewandelt wird, die von $R_4$ verschiedene Gruppe $X_1$ abspaltet und durch Wasserstoff ersetzt, und/oder die von Y verschiedene Gruppe Y' in die Gruppe Y umwandelt, gegebenenfalls weitere an funktionelle Gruppen gebundene Schutzgruppen abspaltet und durch Wasserstoff ersetzt, wobei die Ausgangsstoffe der Formel II bis X, sofern sie salzbildende Eigenschaften aufweisen, auch in Form ihrer Salze verwendet werden können, und die Gruppen R, $R_1$, $R_2$, $R_3$, $R_4$, X, Z, $Alk_1$, $Alk_2$, Y und $Ar_1$ die unter der Formel I angegebenen Bedeutungen haben, und, wenn

erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden basischen Eigenschaften in ein Salz umwandelt, und/oder, wenn erwünscht, ein erhaltenes Racematgemisch in die reinen Racemate bzw. Diastereomeren und/oder ein erhaltenes Racemat in die optischen Antipoden auftrennt.

Ueblicherweise werden die in der Verfahrensvariante a) verwendeten Ausgangsstoffe der Formel II *in situ* gebildet und der verfahrensgemässe Ringschluss kann unter den Reaktionsbedingungen für die Herstellung des Ausgangsmaterials stattfinden. So kann man die Ausgangsstoffe der Formel II und unter den Reaktionsbedingungen üblicherweise auch die entsprechenden Endprodukte der Formel I erhalten, indem man

aa) eine Verbindung der Formel III oder ein reaktionsfähiges funktionelles Derivat davon mit einer Verbindung der Formel

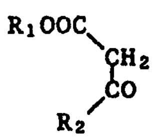

(XI),

einer Verbindung der Formel

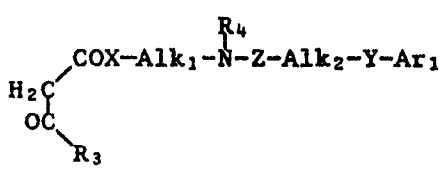

(XII),

und Ammoniak umsetzt, oder

ab) eine Verbindung der Formel III oder ein reaktionsfähiges funktionelles Derivat davon mit einer Verbindung der Formel

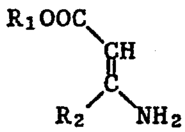

(XIII)

und einer Verbindung der Formel XII umsetzt, oder

ac) eine Verbindung der Formel III oder ein reaktionsfähiges funktionelles Derivat davon mit einer Verbindung der Formel

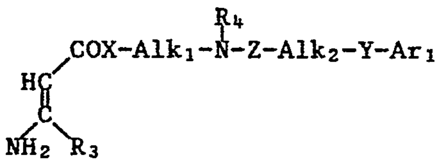

(XIV)

und einer Verbindung der Formel XI oder XIII umsetzt, oder

ad) Ammoniak mit einer Verbindung der Formel

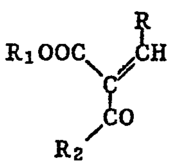

(XV)

und einer Verbindung der Formel XII umsetzt, oder

ae) Ammoniak mit einer Verbindung der Formel

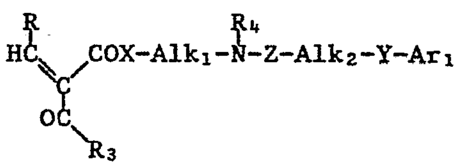

(XVI)

und einer Verbindung der Formel XI oder XIII umsetzt, oder

af) Ammoniak mit einer Verbindung der Formel

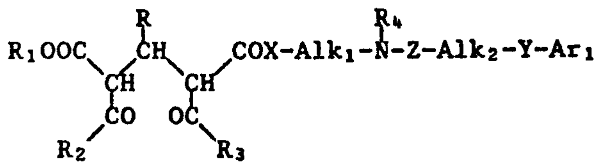

(XVII)

umsetzt, oder

ag) eine Verbindung der Formel XIII mit einer Verbindung der Formel XVI oder der Formel

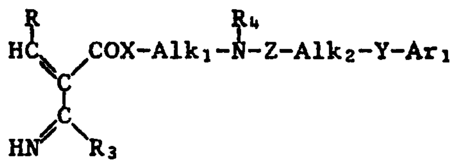

(XVIII)

umsetzt, oder

ah) eine Verbindung der Formel XIV mit einer Verbindung der Formel XV oder mit einer Verbindung der Formel

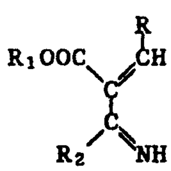

(XIX)

umsetzt. Dabei können die Verbindungen der Formeln XI bis XIX auch in Form von Tautomeren davon oder in Form von Tautomerengemischen verwendet werden; Ausgangsstoffe der obigen Formeln mit salzbildenden Eigenschaften können auch in Form von Salzen verwendet werden. Ferner haben in den obengenannten Verbindungen die Gruppen R, $R_1$, $R_2$, $R_3$, $R_4$, X, Z, $Alk_1$, $Alk_2$, Y und $Ar_1$ die im Zusammenhang mit der Formel I gegebenen Bedeutungen.

Reaktionsfähige funktionelle Derivate des Aldehyds der Formel III sind u.a. die entsprechenden Acetale, d.h. die entsprechenden R-Di-(veräthertes Hydroxy)-methyl-Verbindungen, wie Diniederalkyl-, z.B. Dimethyl- oder Diäthylacetale oder Acylale, z.B. die entsprechenden Di-Acyloxymethyl-Verbindungen, wie Diniederalkanoylacylale, z.B. Diacetylacylale, oder die entsprechenden Dihalogen-, z.B. Dichlor- oder Dibrom-Verbindungen, ferner Additionsverbindungen, wie solche mit einem Alkalimetall-, z.B. Kaliumhydrogensulfit.

Das für die vorstehend beschriebenen Ringschlussreaktionen verwendete Ammoniak kann auch in Form eines diese Verbindung in situ abgebenden Mittels, z.B. in Form eines Ammoniumsalzes, wie Ammoniumacetat oder Ammoniumhydrogencarbonat, verwendet werden.

Bei der Ringschlussreaktion a), sowie den Kondensationsreaktionen aa) bis ah) zur Herstellung des üblicherweise in situ gebildeten Ausgangsmaterials für die Ringschlussreaktion, handelt es sich um Varianten der Dihydropyridinsynthese von Hantzsch. Bei der Variante aa) werden insgesamt drei Moleküle Wasser abgespalten; bei weiteren Varianten tritt teilweise an die Stelle der Wasserabspaltung eine Additionsreaktion, d.h. die Wasserabspaltung erfolgt schon bei der Herstellung von einem oder von zwei Ausgangsstoffen. Bei der Umsetzung von Verbindungen der Formel III mit Verbindungen der Formeln XIV und XIII gemäss der Stufe ac), von Verbindungen der Formel XIII mit Verbindungen der Formel XVIII gemäss der Stufe ag) oder von Verbindungen der Formel XIV mit Verbindungen der Formel XIX gemäss Stufe ah) wird zusätzlich zu bzw. anstelle von Wasser Ammoniak abgespalten. Falls nach der Variante aa) Verbindungen der Formel I hergestellt werden sollen, in denen sich $R_2$ und $R_3$ voneinander unterscheiden, können Nebenprodukte entstehen, die in 2- und 6-Stellung die gleichen Substituenten enthalten. Durch nicht-gleichzeitiges Zusammengeben der Reaktionsteilnehmer kann die Bildung solcher Nebenprodukte indessen herabgesetzt werden, indem man einen bestimmten Reaktionsverlauf fördert, der in situ gemäss einer

anderen Variante verläuft, da entsprechend der gestaffelten Zugabe der Reaktionskomponenten z.B. zunächst eine Verbindung der allgemeinen Formel XIII oder der Formel XIV entstehen kann.

Die verfahrensgemässen Ringschluss- bzw. Kondensationsreaktionen werden in an sich bekannter Weise durchgeführt, falls notwendig, in Gegenwart eines Kondensationsmittels, insbesondere eines basischen Kondensationsmittels, wie eines Ueberschusses einer basischen Reaktionskomponente oder einer zusätzlichen, z.B. organischen Base, wie Piperidin oder Aethyl-diisopropyl-amin, oder eines Metallalkoholats, wie Alkalimetall-niederalkanolats, und/oder eines geeigneten Dehydratisierungs- oder Wasser-aufnehmenden Mittels, ferner üblicherweise in Gegenwart eines inerten organischen Lösungsmittels und bei Reaktionstemperaturen im Bereich von etwa Raumtemperatur bis etwa 150°C, insbesondere bei Siedetemperatur des Lösungsmittels. Gegebenenfalls erfolgt die Umsetzung in einer Inertgas-, z.B. Stickstoffatmosphäre, und/oder, z.B. bei Verwendung eines niedrigsiedenden Lösungsmittels und/oder von Ammoniak, im geschlossenen Gefäss unter erhöhtem Druck.

Die in den Verfahrensvarianten verwendeten Ausgangsstoffe sind bekannt oder können nach an sich bekannten Verfahren hergestellt werden.

So lassen sich z.B. Ausgangsstoffe der Formel XII durch Umsetzung von Verbindungen der Formel

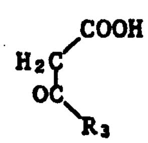

(XIIa)

mit Verbindungen der Formel

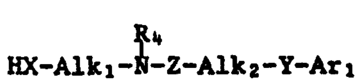

(XIIb)

auf übliche Weise herstellen. Anstelle von Carbonsäuren der Formel XIIa können auch funktionelle Derivate davon, wie entsprechende Carbonsäureanhydride, insbesondere gemischte Anhydride, wie solche mit Niederalkancarbonsäuren, etwa Ameisensäure, ferner Säurehalogenide, z.B. entsprechende Chloride, Bromide, ferner Säureazide, weiter aktivierte Ester, z.B. Cyanmethylester verwendet werden. Diese können, gegebenenfalls in Gegenwart von Kondensationsmitteln, durch Umsetzung mit einer Verbindung der Formel XIIb, freie Carbonsäuren der Formel XIIa auch durch Umsetzen mit Verbindungen der Formel XIIb, worin anstelle der Gruppe HX- die Azidogruppe steht, zu Verbindungen der Formel XII umgesetzt werden. Carbonsäuren der Formel XIIa können auch als Salze, insbesondere als Alkalimetall- oder Erdalkalimetallsalze mit reaktionsfähigen Estern von Alkoholen der Formel XIIb, worin X für Sauerstoff steht, wie entsprechenden Halogeniden, z.B. Chloriden, Bromiden oder Iodiden, oder organischen Sulfonsäureestern, z.B. Niederalkansulfonsäure- oder Arensulfonsäureestern, wie Methansulfonsäure- bzw. p-Toluolsulfonsäureestern, zu entsprechenden Carbonsäureestern umgesetzt werden, oder man hydrolysiert entsprechende hydrolysierbare Iminoester, wie entsprechende Iminoniederalkylester, zu den Estern. Iminoester dieser Art sind beispielsweise aus den Verbindungen der Formel XIIa entsprechenden Nitrilen der Formel

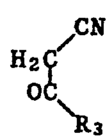

(XIIc)

durch Umsetzung mit Verbindungen der Formel XIIb, worin X für Sauerstoff steht, in Gegenwart eines sauren Kondensationsmittels, etwa Chlorwasserstoff, in einem geeigneten Lösungsmittel, etwa eines solchen inerten Charakters, wie eines Aromaten, z.B. Benzol, auf übliche Weise zugänglich.

Verbindungen der Formel XIIb wiederum sind auf an sich bekannte Weise durch Umsetzung von Verbindungen der Formel

$HX-Alk_1-OH$ (XIId),

worin die Hydroxygruppe in reaktionsfähiger veresterter Form, wie etwa als Halogen, z.B. Chlor, Brom oder Iod oder eine Sulfonyloxygruppe, z.B. eine Arylsulfonyloxy- wie p-Toluolsulfonyloxygruppe, vorliegen kann, mit Verbindungen der Formel

$$H-\overset{R_4}{\overset{|}{N}}-Z-Alk_2-Y-Ar_1$$ (XIIe),

zweckmässigerweise in Gegenwart eines Kondensationsmittels, etwa eines solchen basischen Charakters, wie eines Oxids, Hydroxids oder Carbonats eines Alkalimetall- oder Erdalkalimetalls, wie Natriumhydroxid oder Calciumcarbonat, und üblicherweise in Gegenwart eines Lösungsmittels, etwa eines Niederalkanols, wie Aethanol, bei erhöhter oder erniedrigter Temperatur, zugänglich. Verbindungen der Formel XIIe können auch in Form ihrer Metallderivate eingesetzt werden, worin das am Stickstoff stehende Wasserstoffatom durch ein geeignetes Metall, etwa Lithium oder Kalium, ersetzt ist. In solchen Fällen erfolgt die beschriebene Umsetzung mit Verbindungen der Formel XIId in einem inerten wasserfreien Lösungsmittel, etwa einem solchen ätherartigen Charakters, wie Tetrahydrofuran, oder einem aromatischen Lösungsmittel, etwa Toluol, zweckmässigerweise unter einem Schutzgas, wie Stickstoff.

Der Formel XIIe entsprechende Metallverbindungen sind in üblicher Weise, z.B. durch Umsetzung mit einer geeigneten Alkalimetall-organischen Verbindung, etwa Butyllithium in einem wasserfreien Lösungsmittel, wie Tetrahydrofuran, zweckmässigerweise unter einem Schutzgas, z.B. Argon, zugänglich, wobei die im Reaktionsgemisch enthaltene Metallverbindung, ohne diese zu isolieren, für die oben beschriebene Umsetzung verwendet werden kann.

Verbindungen der Formel XIIe wiederum sind auf an sich bekannte Weise herstellbar, etwa durch Umsetzung einer Verbindung der Formel

$HO-Z-Alk_2-Y-Ar_1$ (XIIf)

oder eines reaktionsfähigen Derivats davon, mit einem Amin der Formel

$R_4-NH_2$ (XIIg).

Liegt Z in der Formel XIIf als Gruppe -(C=O)- vor, so kann die zugrundeliegende Carbonsäure der Formel XIIf auch als funktionelles Derivat, etwa als entsprechendes Carbonsäureanhydrid, insbesondere gemischtes Anhydrid, wie eines mit Niederalkancarbonsäuren, etwa Ameisensäure, ferner als Säurehalogenid, z.B. als entsprechendes Chlorid, Bromid, ferner Säureazid, weiter als aktivierte Ester, z.B. Cyanmethylester, eingesetzt werden. Diese können, gegebenenfalls in Gegenwart von Kondensationsmitteln, etwa von solchen basischen Charakters, z.B. von tertiären Aminen wie N-Aethyl-N,N-diisopropylamin oder einem Ueberschuss des umzusetzenden Amins der Formel XIIg mit einem Amin der Formel XIIg in üblicher Weise zu einer Verbindung der Formel XIIe, worin Z die Gruppe -(C=O)- ist, umgesetzt werden.

Liegt Z in der Formel XIIf als einfache Bindung vor, so kann der entsprechende Alkohol auch in Form eines reaktionsfähigen Esters, etwa als Halogenid, wie Chlorid oder Bromid, oder als Sulfonsäureester wie Methan- oder p-Toluolsulfonsäureester vorliegen, der mit einem Amin der Formel XIIg üblicherweise in Gegenwart eines Kondensationsmittels, etwa eines solchen basischen Charakters, wie eines Carbonats, Oxids oder Hydroxids eines Alkalimetalls oder Erdalkalimetalls, z.B. Natriumcarbonat oder -hydroxid, verwendet wird. Solche Umsetzungen werden zweckmässigerweise in einem inerten Lösungsmittel vorgenommen, z.B. einem solchen aromatischen Charakters, wie Benzol oder Toluol für die Reaktion von reaktionsfähigen Carbonsäurederivaten mit einem Amin der Formel XIIg, oder einem Niederalkanol, wie Aethanol, für die Umsetzung von reaktionsfähigen veresterten Alkanolen der Formel XIIf mit einem Amin der Formel XIIg. Letzteres kann auch als N-Metallderivat, z.B. als Alkalimetallderivat, wie ein solches mit Lithium, eingesetzt werden, wobei man solche Reaktionen üblicherweise in einem ätherartigen Lösungsmittel, etwa Di-n-butyläther, und unter einem Schutzgas, wie Stickstoff oder Argon, vornimmt.

Die Umsetzung von freien Carbonsäuren der Formel XIIa mit Verbindungen der Formel XIIb erfolgt vorteilhaft in Gegenwart eines sauren, die Wasserabspaltung fördernden Katalysators, wie einer Protonensäure, z.B. Chlorwasserstoff-, Bromwasserstoff-, Schwefel-, Phosphor- oder Borsäure, Benzolsulfon- oder Toluolsulfonsäure, oder einer Lewissäure, z.B. von Bortrifluorid-Aetherat, in einem Ueberschuss des eingesetzten Alkohols und/oder in einem inerten Lösungsmittel, erforderlichenfalls unter destillativer, z.B. azeotroper, Entfernung des bei der Reaktion freigesetzten Wassers. Weiter kann man die Umsetzungen auch in Gegenwart von wasserbindenden Kondensationsmitteln, wie geeignet substituierten Carbodiimiden, z.B. N,N'-Diäthyl-, N,N'-Dicyclohexyl- oder N-Aethyl-N'-(3-dimethylaminopropyl)-carbodiimid, in inerten organischen Lösungsmitteln durchführen. Gemischte Anhydride, insbesondere Säurehalogenide, werden beispielsweise in Gegenwart säurebindender Mittel, z.B. organischer, insbesondere tertiärer Stickstoffbasen, wie Triäthylamin, Aethyldiisopropylamin oder Pyridin, oder auch anorganischer Basen, z.B. von Alkalimetall- oder Erdalkalimetallhydroxiden oder -carbonaten, wie Natrium-, Kalium- oder Calciumhydroxid bzw. -carbonat, mit Alkoholen oder mit Alkoholaten, z.B. Alkalimetall-niederalkanolaten, umgesetzt.

Die Umsetzungen von reaktionsfähigen Estern, z.B. Cyanmethyl- oder Pentachlorphenylestern, mit Verbindungen der Formel XIIb werden beispielsweise in einem gegenüber den Reaktionsteilnehmern inerten Lösungsmittel im Temperaturbereich von etwa 0°C bis etwa 120°C, vorzugsweise bei Raumtemperatur bis etwa 60°C, durchgeführt.

Die Hydrolyse von Imidoesterausgangsstoffen erfolgt beispielsweise mittels wasserhaltiger Mineralsäuren, wie Salzsäure oder Schwefelsäure, wobei man z.B. die bei der Addition von Chlorwasserstoff an Nitrile und Umsetzung mit wasserfreien Alkoholen, insbesondere unsubstituierten oder substituierten Niederalkanolen, erhaltenen Iminoester-salze, z.B. -hydrochloride, nach Zusatz von Wasser direkt zu den entsprechenden Estern hydrolysieren kann. Man kann z.B. auch aus einem Gemisch von Nitril, Alkohol und Schwefelsäure mit geeignetem Wassergehalt ohne Isolierung des in situ entstandenen Imidoesters die gewünschte Esterverbindung der Formel XI erhalten.

Ausgangsstoffe der Formeln XIV, XVI, XVII und XVIII können ausgehend von entsprechenden Ausgangsmaterialien auf analoge und übliche Weise hergestellt werden.

Ausgangsstoffe der Formel IV werden analog wie bei der Herstellung von Ausgangsstoffen der Formel II beschrieben in situ gebildet, und der verfahrensgemässe Ringschluss zu den Endprodukten der Formel I kann unter den für die Herstellung des Ausgangsmaterials gegebenen Bedingungen im gleichen Reaktionsansatz erfolgen. Dementsprechend sind Ausgangsstoffe der Formel IV durch Umsetzung von Verbindungen der Formel XII mit solchen der Formel XI und Ammoniak, oder von Verbindungen der Formel XII mit solchen der Formel XIII, oder von Verbindungen der Formel XIV mit solchen der Formel XI oder XIII zugänglich, wobei solche Umsetzungen üblicherweise in einem geeigneten Lösungsmittel, etwa einem Niederalkanol, wie Aethanol, gegebenenfalls bei erhöhter oder erniedrigter Temperatur, zweckmässigerweise unter einem Schutzgas, wie Stickstoff, durchgeführt werden.

Ausgangsstoffe der Formel V können entsprechend dem bzw. den in ihnen enthaltenen Rest(en) $Ac^o$ beispielsweise Carbonsäuren ($Ac^o$ ist Carboxyl), Carbonsäureanhydride, insbesondere gemischte Anhydride, wie Säurehalogenide, z.B. -chloride oder -bromide oder -azide ($Ac^o$ ist Halogencarbonyl, z.B. Chlor- oder Bromcarbonyl oder Azidocarbonyl), weiter aktivierte Ester, z.B. Cyanmethylester ($Ac^o$ ist Cyanmethoxycarbonyl) sein; diese können, gegebenenfalls in Gegenwart von Kondensationsmitteln, durch Behandeln mit einem entsprechenden Alkohol, in die entsprechenden Ester umgewandelt werden, beispielsweise zur Umwandlung der Gruppe $Ac^o$ in eine der Gruppe $-COOR_1$ entsprechende Estergruppe durch Umsetzung mit einem der Bedeutung von $R_1$ entsprechenden Niederalkanol, oder einem reaktionsfähigen Derivat davon, z.B. einem entsprechenden Alkoholat, freie Carbonsäuren auch durch Umsetzen mit geeigneten Diazo-Verbindungen, wie Diazoniederalkanen, wobei Verbindungen der Formel I entstehen, welche die Gruppe $-COOR_1$ enthalten.

Zur Umwandlung der Gruppe $Ac_1^o$ in die Gruppe der oben definierten Formel Va setzt man einen Ausgangsstoff der Formel V, worin $Ac_1^o$ Carboxyl, der Rest eines Anhydrids, insbesondere eines gemischten Anhydrids, wie Säurehalogenids, z.B. -chlorids oder -bromids, oder eines Azids, oder eines aktivierten Esters, wie eines Cyanmethylesters ist, mit einer Verbindung der Formel XIIb in üblicher Weise um. Carbonsäureester der angegebenen Art, worin X für Sauerstoff steht, können ebenfalls erhalten werden, wenn als Ausgangsstoffe Salze, insbesondere Alkalimetall- oder Erdalkalimetallsalze der freien Carbonsäuren verwendet und diese mit reaktionsfähigen Estern von den Verbindungen der Formel XIIb entsprechenden Alkoholen, worin X Sauerstoff ist, wie entsprechenden Halogeniden, z.B. Chloriden, Bromiden oder Iodiden, oder organischen Sulfonsäureestern, z.B. Niederalkansulfonsäure- oder Arensulfonsäureestern, wie Methansulfonsäure- bzw. p-Toluolsulfonsäureestern, behandelt werden, oder wenn man entsprechende hydrolysierbare Iminoester, wie entsprechende Iminoniederalkylester, zu den Estern hydrolysiert.

Iminoester dieser Art sind z.B. aus Ausgangsstoffen der Formel V, worin $Ac^o$ die Cyangruppe darstellt, durch Umsetzung mit einem der Bedeutung von $R_1$ entsprechenden Niederalkanol und/oder einem Alkohol der Formel XIIb, worin X Sauerstoff ist, in Gegenwart eines sauren Kondensationsmittels, wie Chlorwasserstoff oder konz. Schwefelsäure zugänglich.

Die Umsetzung von freien Carbonsäuren mit Alkoholen, wie Niederalkanolen oder mit Verbindungen der Formel XIIb, erfolgt vorteilhaft auf die gleiche Weise, die vorstehend für die Umsetzung von Carbonsäuren der Formel XIIa mit Verbindungen der Formel XIIb beschrieben ist.

Die Umsetzungen von der Formel V entsprechenden reaktionsfähigen Estern, z.B. Cyanmethyl-, Benztriazol-1-yl- oder Pentachlorphenylestern, mit der Bedeutung von $R_1$ entsprechenden Niederalkanolen bzw. mit Verbindungen der Formel XIIb werden beispielsweise in einem gegenüber den Reaktionsteilnehmern inerten Lösungsmittel im Temperaturbereich von etwa 0°C bis etwa 120°C, vorzugsweise bei Raumtemperatur bis etwa 60°C, durchgeführt.

Auch die Hydrolyse von Imidoesterausgangsstoffen erfolgt beispielsweise wie vorstehend im Zusammenhang mit der Verfahrensvariante a) beschrieben.

Ausgangsstoffe der Formel V mit freier Carboxylgruppe $Ac^o$ können z.B. erhalten werden, indem man den entsprechenden 2-Cyanäthylester herstellt, wobei man z.B. in einem der vorstehend beschriebenen Verfahren ab) oder ag) eine Verbindung der Formel XIII einsetzt, in der anstelle der Gruppe $-COOR_1$ eine 2-Cyanäthoxycarbonylgruppe steht; z.B. kann man einen die Gruppe $R_2$ enthaltenden 3-Aminocrotonsäure-2-cyanäthylester mit den übrigen Reaktionskomponenten umsetzen und anschliessend die so erhaltene 2-Cyanäthylester-Verbindung unter milden Bedingungen, z.B. mittels wässrigem oder wässerig-niederalkanolischem 1-n. Natriumhydroxid bei Raumtemperatur, zur freien Carbonsäure spalten. Letztere kann, falls notwendig, in an sich bekannter Weise in die gewünschten reaktionsfähigen funktionellen Derivate übergeführt werden.

Die als Ausgangsstoffe für die Verfahrensvariante c) ebenfalls in Betracht kommenden Nitrilverbindungen der Formel V können z.B. analog zu einer der Verfahrensvarianten aa) bis ah) hergestellt werden, indem man Ausgangsstoffe verwendet, die anstelle des Restes $-COOR_1$ eine Cyangruppe enthalten, wie beispielsweise anstelle einer Verbindung der Formel XIII die Gruppe $R_2$ enthaltendes 3-Aminocrotononitril.

Die für die Verfahrensvariante d) benötigten Ausgangsstoffe der Formel VI können auf an sich bekannte Weise hergestellt werden, z.B. analog den in den Verfahrensstufen aa) bis ah) beschriebenen Umsetzungen, wobei anstelle der Verbindungen XII, XIV, XVI, XVII oder XVIII solche Ausgangsmaterialien eingesetzt werden, die anstelle der Gruppe der Formel

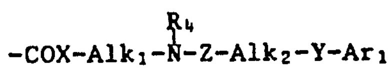

(Va)

die Gruppe der Formel $-COX-Alk_1-OH$ (VIb) bzw. einen reaktionsfähigen Ester davon aufweisen. Reaktionsfähige Ester sind z.B. mit einer Halogenwasserstoffsäure, z.B. Salzsäure, oder einer organischen Sulfonsäure, wie einer Arylsulfonsäure, z.B. p-Toluolsulfonsäure gebildete Ester, in denen die Hydroxygruppe also z.B. durch Halogen, wie Chlor oder Brom, oder z.B. durch Arylsulfonyloxy, wie p-Toluolsulfonyloxy ersetzt ist. So kann z.B. ein Ausgangsstoff der Formel

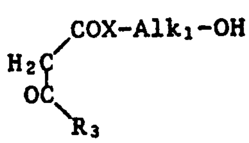

(VIc)

analog den für die Herstellung von Ausgangsstoffen der Formel XII beschriebenen Methoden, z.B. durch Umsetzung von Verbindungen der Formel XIIa mit Verbindungen der Formel HX-$Alk_1$-OH (XIId) oder einem reaktionsfähigen Ester, etwa einem Halogenid, wie Bromid oder Iodid, wie beschrieben, erhalten werden. Oder man setzt ein Nitril der Formel XIIc mit einer Verbindung der Formel XIId, worin X Sauerstoff ist, in Gegenwart eines sauren Kondensationsmittels, etwa Chlorwasserstoff, zum entsprechenden Iminoester-Salz um, der dann mittels Wasser im sauren Medium, z.B. wie oben beschrieben, zum entsprechenden Carbonsäureester hydrolysiert wird.

Die obigen Reaktionen und ebenso die Verfahrensvariante d) können unter an sich bekannten Reaktionsbedingungen durchgeführt werden, in Ab- oder üblicherweise Anwesenheit von Lösungs- oder Verdünnungsmitteln, je nach Art der Reaktion und/oder Reaktionsteilnehmer bei erniedrigter oder erhöhter Temperatur, z.B. im Temperaturbereich von etwa -10°C bis etwa 150°C, unter atmosphärischem Druck oder in einem geschlossenen Gefäss, gegebenenfalls unter Druck, und/oder in einer inerten Atmosphäre, z.B. unter einer Stickstoffatmosphäre.

Die für die Verfahrensvariante e) benötigten mittels Reduktion in die Gruppe $Alk_1$ bzw. $Alk_2$ überführbaren Gruppen $Z_1$ bzw. $Z_2$ enthalten C-C-Doppel- und/oder Dreifachbindungen und/oder Carbonyl- bzw. Thiocarbonylgruppen im entsprechenden Alkylenrest.

Die Reduktion von ungesättigten Gruppen zur Kohlenstoff-Kohlenstoff-Einfachbindung erfolgt beispielsweise mittels aktiviertem Wasserstoff, wie Wasserstoff in Gegenwart eines Hydrierkatalysators, z.B. eines Nickel-, Platin- oder Palladiumkatalysators, während die Reduktion von Carbonylgruppen zur Methylengruppe mittels Wasserstoff in Gegenwart z.B. eines Kupferchromit-Katalysators oder nach der Methode von Clemmensen, z.B. mittels gegebenenfalls analgamiertem Zink in einer Mineralsäure, etwa Salzsäure, erfolgen kann. Die Reduktion von Thiocarbonylgruppen kann auf ähnliche Weise erfolgen, z.B. unter Verwendung eines schwefelfesten Katalysators. Die Reduktion der vorstehend genannten Gruppen kann ferner mittels eines

Hydridreduktionsmittels, z.B. Natriumborhydrid oder Diboran, erfolgen. Bei diesen Reduktionen muss, sofern erwünscht, Sorge dafür getragen werden, dass andere gegen Reduktion empfindliche Gruppen, z.B. ungesättigte Gruppen oder Nitrogruppen, nicht angegriffen werden, z.B. durch Auswahl des geeigneten Reduktionsmittels, dessen für die durchzuführende Reduktion erforderliche Dosierung und/oder geeignete Verfahrensbedingungen, z.B. erhöhte oder erniedrigte Temperatur und/oder durch Anwendung eines geeigneten Lösungsmittels. Diese Umsetzungen werden in an sich bekannter Weise durchgeführt, üblicherweise in Anwesenheit von Lösungs- und Verdünnungsmitteln, je nach Art der Reduktion und/oder Reaktionsteilnehmer bei erniedrigter oder erhöhter Temperatur, z.B. im Temperaturbereich von etwa -15°C bis etwa 120°C unter normalem Druck oder im geschlossenen Gefäss, gegebenenfalls unter Druck und/oder unter einem Schutzgas, etwa Stickstoff.

Ausgangsstoffe der Formel VII können nach an sich bekannten Methoden hergestellt werden, z.B. analog den unter a) bis d) beschriebenen Verfahren. So kann man z.B. analog den in den Stufen aa) bis ah) beschriebenen Reaktionsschritten verfahren, wenn man anstelle der Verbindungen XII, XIV, XVI, XVII oder XVIII solche Verbindungen einsetzt, die anstelle der oben definierten Gruppe Va die Gruppe der Formel

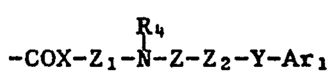

(VIIa)

enthalten.

So kann z.B. ein Ausgangsstoff der Formel

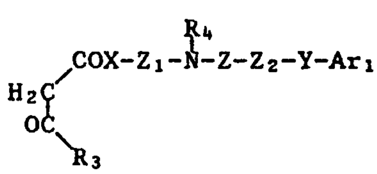

(VIIb)

analog den für die Herstellung von Verbindungen der Formel XII beschriebenen Methoden, etwa ausgehend von Verbindungen der Formel XIIa oder reaktionsfähigen Derivaten davon, z.B. Anhydriden, Säurehalogeniden, etwa Chloriden, Aziden, gemischten Estern wie Cyanmethylestern oder Pentachlorphenolestern, verwendet werden. Carbonsäuren der Formel XIIa können auch als Salze, insbesondere als Alkalimetall- oder Erdalkalimetallsalze mit reaktionsfähigen Estern der Formel

$$HO-Z_1-\overset{R_4}{\overset{|}{N}}-Z-Z_2-Y-Ar_1 \qquad (VIIc),$$

wie entsprechenden Halogeniden, z.B. Chloriden, Bromiden oder Iodiden, oder organischen Sulfonsäureestern, etwa solchen mit Niederalkansulfonsäuren, wie Methansulfonsäure, oder Arylsulfonsäuren, wie Benzolsulfonsäure, zu entsprechenden Carbonsäureestern umgesetzt werden. Alkohole der Formel VIIc können andererseits mit Nitrilen der Formel XIIc in üblicher Weise in Gegenwart saurer Kondensationsmittel, wie einer Mineralsäure, etwa Chlorwasserstoff- oder Schwefelsäure, zu den Salzen entsprechender Iminoester umgesetzt werden, die dann mittels Wasser, wie beschrieben, zu den Carbonsäureestern hydrolysiert werden. Diese Umsetzungen werden unter üblichen und an sich bekannten Reaktionsbedingungen vorgenommen. Ausgangsstoffe der Formel VIIc wiederum sind, z.B. durch Umsetzung von Verbindungen der Formel $HO-Z_1-A$ (VIId), worin A eine geeignete Abgangsgruppe, z.B. Halogen, wie Chlor, Brom oder Iod oder eine Sulfonyloxy- wie p-Toluolsulfonyloxygruppe ist, mit Verbindungen der Formel XIIe auf übliche Weise zugänglich.

Falls in den für die Verfahrensvariante f) benötigten Ausgangsstoffen der Formel IX die Gruppe Z die Gruppe -(C=O)- bedeutet, so kann die entsprechende Carbonsäure auch als funktionelles Derivat, etwa als entsprechendes Carbonsäureanhydrid, insbesondere gemischtes Anhydrid, wie eines mit Niederalkancarbonsäuren, etwa Ameisensäure,

ferner als Säurehalogenid, z.B. als entsprechendes Chlorid, Bromid, ferner Säureazid, weiter als aktivierter Ester, z.B. Cyanmethylester, eingesetzt werden.

Die Umsetzung von freien Carbonsäuren der Formel IX mit Verbindungen der Formel VIII erfolgt vorteilhaft auf die gleiche Weise, die vorstehend für die Umsetzung von Carbonsäuren der Formel XIIa mit Verbindungen der Formel XIIb beschrieben ist.

Die Umsetzungen von reaktionsfähigen Estern, z.B. Cyanmethyl- oder Pentachlorphenylestern der Formel IX mit Verbindungen der Formel VIII werden beispielsweise in einem gegenüber den Reaktionsteilnehmern inerten Lösungsmittel im Temperaturbereich von etwa 0°C bis etwa 120°C, vorzugsweise bei Raumtemperatur bis etwa 60°C, durchgeführt.

Wenn Z in der Formel IX eine einfache Bindung bedeutet, so kann die Hydroxygruppe auch in reaktionsfähiger veresterter Form, wie etwa als Halogen, z.B. Chlor, Brom oder Iod oder als Sulfonyloxygruppe, z.B. als Arylsulfonylgruppe, wie als p-Toluolsulfonyloxygruppe vorliegen, die mit einem Amin der Formel VIII zweckmässigerweise in Gegenwart eines Kondensationsmittels, etwa eines solchen basischen Charakters, wie eines Oxids, Hydroxids oder Carbonats eines Alkalimetall- oder Erdalkalimetalls, wie Natriumhydroxid oder Calciumcarbonat, und üblicherweise in Gegenwart eines Lösungsmittels, etwa eines Niederalkanols, wie Aethanol, bei erhöhter oder erniedrigter Temperatur umgesetzt wird. Verbindungen der Formel VIII können auch in Form ihrer Metallderivate eingesetzt werden, worin das am Stickstoff stehende Wasserstoffatom durch ein geeignetes Metall, etwa Lithium oder Kalium ersetzt ist. In solchen Fällen erfolgt die beschriebene Umsetzung mit Verbindungen der Formel IX, worin anstelle der Hydroxygruppe z.B. Halogen, wie etwa Chlor, steht, in einem inerten wasserfreien Lösungsmittel, etwa eines solchen ätherartigen Charakters, wie Tetrahydrofuran, oder eines aromatischen Lösungsmittels, etwa Toluol, zweckmässigerweise unter einem Schutzgas, wie Stickstoff.

Der Formel VIII entsprechende Metallverbindungen sind in üblicher Weise, z.B. durch Umsetzung mit einer geeigneten Alkalimetall-organischen Verbindung, etwa Butyllithium in einem wasserfreien Lösungsmittel, wie Tetrahydrofuran, zweckmässigerweise unter einem Schutzgas, z.B. Argon, zugänglich, wobei die im Reaktionsgemisch enthaltene Metallverbindung für die oben beschriebene Umsetzung verwendet werden kann, ohne dass sie isoliert werden müsste.

Ausgangsstoffe der Formel VIII können, wie bei der Herstellung von Ausgangsstoffen der Formel II beschrieben, analog in situ gebildet werden, wobei der Ringschluss zu Verbindungen der Formel VIII unter den gegebenen Bedingungen im gleichen Reaktionsansatz erfolgen kann. Dementsprechend sind Ausgangsstoffe der Formel VIII beispielsweise durch Umsetzung von Verbindungen der Formel XV oder XIX mit Verbindungen der Formel

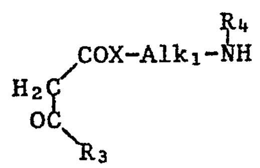

(XIIh)

oder der Formel

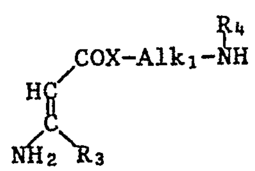

(XIVa)

mit Ammoniak zugänglich; oder man kann Verbindungen der Formel XI oder XIII mit solchen der Formel

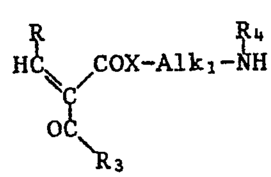

(XVIa)

oder der Formel

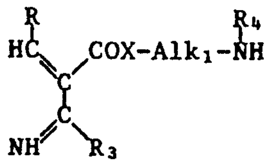

(XVIIIa)

oder Verbindungen der Formel

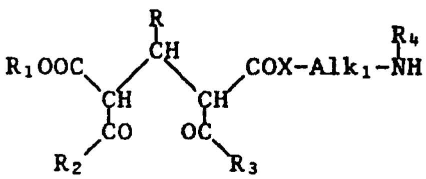

(XVIIa)

jeweils mit Ammoniak zu Ausgangsstoffen der Formel VIII umsetzen, wobei solche Umsetzungen üblicherweise in einem geeigneten Lösungsmittel, etwa einem Niederalkanol, wie Aethanol, gegebenenfalls bei erhöhter oder erniedrigter Temperatur, zweckmässigerweise unter einem Schutzgas, wie Stickstoff, durchgeführt werden. Anstatt den 1,4-Dihydropyridinring in situ zu bilden, kann man aber auch von fertig gebildeten Carbonsäuren der Formel

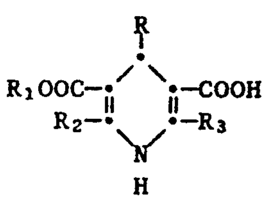

(VIIIa),

ausgehen und diese mit Verbindungen der Formel $HX-Alk_1-NHR_4$ (VIIIb) umsetzen. Anstelle von Carbonsäuren der Formel (VIIIa) können auch funktionelle Derivate davon, wie entsprechende Carbonsäureanhydride, insbesondere gemischte Anhydride, wie solche mit Niederalkancarbonsäuren, etwa Ameisensäure, ferner Säurehalogenide, z.B. entsprechende Chloride, Bromide, ferner Säureazide, weiter aktivierte Ester, z.B. Cyanmethylester verwendet werden. Diese können, gegebenenfalls in Gegenwart von Kondensationsmitteln, durch Umsetzung mit einer Verbindung der Formel VIIIb, freie Carbonsäuren der Formel VIIIa auch durch Umsetzung mit Verbindungen der Formel VIIIb, worin anstelle der Gruppe HX- die Azidogruppe steht, zu Verbindungen der Formel VIII umgesetzt werden. Carbonsäuren der Formel VIIIa können auch als Salze, insbesondere als Alkalimetall- oder Erdalkalimetallsalze mit reaktionsfähigen Estern von Alkoholen der Formel VIIIb, worin X für Sauerstoff steht, wie entsprechenden Halogeniden, z.B. Chloriden, Bromiden oder Iodiden, oder Estern von organischen Sulfonsäuren, z.B. Niederalkansulfonsäure- oder Arensulfonsäureestern, wie Methansulfonsäure- bzw. p-Toluolsulfonsäureestern, zu entsprechenden Carbonsäureestern umgesetzt werden, oder man hydrolysiert entsprechende hydrolysierbare Iminoester, wie entsprechende Iminoniederalkylester, beispielsweise mittels wasserhaltiger Mineralsäuren, wie Salzsäure, zu den Estern. Iminoester dieser Art sind beispielsweise aus Nitrilen der Formel

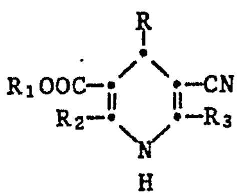

(VIIIc)

durch Umsetzung mit Verbindungen der Formel VIIIb, worin X für Sauerstoff steht, in Gegenwart eines sauren Kondensationsmittels, etwa Chlorwasserstoff, in einem geeigneten Lösungsmittel, etwa einem solchen inerten Charakters, wie einem Aromaten, z.B. Benzol, auf übliche Weise zugänglich.

Die Verfahrensvariante f) sowie die nachfolgend beschriebenen Reaktionen können unter an sich bekannten Reaktionsbedingungen durchgeführt werden, in Ab- oder üblicherweise Anwesenheit von Lösungs- oder Verdünnungsmitteln, je nach Art der Reaktion und/oder Reaktionsteilnehmer bei erniedrigter oder erhöhter Temperatur, z.B. im Temperaturbereich von etwa -10°C bis etwa 150°C, unter atmosphärischem Druck oder in einem geschlossenen Gefäss, gegebenenfalls unter Druck, und/oder in einer inerten Atmosphäre, z.B. unter einer Stickstoffatmosphäre.

Geschützte funktionelle Gruppen gemäss der Verfahrensvariante g) sind z.B. in den aromatischen Resten R und/oder $Ar_1$ enthaltene geschützte Hydroxy- und/oder Aminogruppen, wobei Schutzgruppen abspaltbare und durch Wasserstoff ersetzbare Gruppen darstellen. Eine in die Gruppe Y überführbare Gruppe Y' stellt ein funktionell abgewandeltes und somit geschütztes Derivat der Gruppe -(C=O)- dar, aus welchem die letztere gebildet werden kann. Funktionelle Derivate der Gruppe -(C=O)- sind z.B. die Acetale, wie Diniederalkyl-, z.B. Dimethyl- oder Diäthylacetale, ferner auch cyclische Acetale, wie z.B. das Aethylenacetal, nämlich das entsprechende Dioxolan, ferner Additionsverbindungen, wie solche mit Bisulfiten, etwa einem Alkalimetall-, z.B. Kaliumbisulfit.

Die Abspaltung der Gruppe $X_1$ sowie von Hydroxy- und/oder Aminoschutzgruppen erfolgt mittels Solvolyse, wie Hydrolyse, Alkoholyse oder Acidolyse, oder mittels Reduktion, einschliesslich Hydrogenolyse, wobei die einem funktionell abgewandelten Derivat der Gruppe -(C=O)- entsprechende Gruppe Y' mittels Hydrolyse, insbesondere mittels saurer Hydrolyse in die Gruppe -(C=O)- umgewandelt wird.

Eine besonders geeignete abspaltbare Hydroxy- oder Amino-Schutzgruppe ist in erster Linie eine hydrogenolytisch abspaltbare α-Arylniederalkylgruppe, wie eine gegebenenfalls substituierte 1-Polyphenylniederalkyl- oder 1-Phenylniederalkylgruppe, z.B. Benzhydryl oder Trityl, worin Substituenten, insbesondere des Phenylteils, z.B. Niederalkyl, wie Methyl, oder Niederalkoxy wie Methoxy, sein können, und in erster Linie Benzyl, während eine abspaltbare Gruppe $X_1$ sowie Hydroxy- und/oder Amino-Schutzgruppen einen solvolytisch wie hydrolytisch oder acidolytisch, ferner einen reduktiv, einschliesslich hydrogenolytisch, abspaltbaren Rest, insbesondere einen entsprechenden Acylrest, wie den Acylrest einer organischen Carbonsäure, z.B. Niederalkanoyl, wie Acetyl, oder Aroyl, wie Benzoyl, ferner den Acylrest eines Halbesters der Kohlensäure, wie Niederalkoxycarbonyl, z.B. Methoxycarbonyl, Aethoxycarbonyl oder

tert.-Butyloxycarbonyl, 2-Halogenniederalkoxycarbonyl, z.B. 2,2,2-Trichloräthoxycarbonyl oder 2-Iodäthoxycarbonyl, gegebenenfalls substituiertes 1-Phenylniederalkoxycarbonyl, z.B. Benzyloxycarbonyl oder Diphenylmethoxycarbonyl, oder Aroylmethoxycarbonyl, z.B. Phenacyloxycarbonyl, ferner eine gegebenenfalls substituierte 1-Polyphenyl-niederalkylgruppe, z.B. wie oben angegeben und in erster Linie Trityl darstellen.

Hydrogenolytisch abspaltbare Reste $X_1$, insbesondere gegebenenfalls substituierte 1-Phenylniederalkylgruppen, ferner auch geeignete Acylgruppen, wie gegebenenfalls substituiertes 1-Phenylniederalkoxycarbonyl, sowie in den aromatischen Resten R und/oder $Ar_1$ vorhandene Hydroxy- und/oder Amino-Schutzgruppen dieser Art können durch Behandeln mit katalytisch aktiviertem Wasserstoff, z.B. mit Wasserstoff in Gegenwart eines Katalysators, wie eines geeigneten Edelmetallkatalysators, z.B. Palladium oder Platin abgespalten werden, wobei dafür Sorge zu tragen ist, dass eine der Gruppe $X_1$ entsprechende Phenylniederalkyl-, nämlich Benzylgruppe, sofern erwünscht, nicht abgespalten wird, z.B. durch volumetrische Kontrolle der aufgenommenen Wasserstoffmenge und rechtzeitiges Abbrechen der Hydrierung und/oder durch Wahl eines geeigneten Katalysators und/oder Lösungsmittels.

Hydrolytisch abspaltbare Gruppen $X_1$, wie Acylreste von organischen Carbonsäuren, z.B. Niederalkanoyl, und von Halbestern der Kohlensäure, z.B. Niederalkoxycarbonyl, ferner z.B. Tritylreste, sowie in den Resten R und $Ar_1$ an funktionellen Gruppen, wie an Hydroxy- und/oder Aminogruppen, stehende Schutzgruppen dieser Art können je nach Art der Gruppen durch Behandeln mit Wasser unter sauren oder basischen Bedingungen, z.B. in Gegenwart einer Mineralsäure, wie Chlorwasserstoff- oder Schwefelsäure, dies gilt insbesondere auch für der Bedeutung von Y' entsprechende Acetale, oder eines Alkalimetall- oder Erdalkalimetallhydroxids oder -carbonats oder eines Amins, wie Isopropylamin, abgespalten werden.

Acidolytisch abspaltbare Reste $X_1$ und/oder an funktionellen Gruppen, etwa an Hydroxy und/oder Amino, stehende Schutzgruppen in einem Rest R und/oder $Ar_1$ sind insbesondere gewisse Acylreste von Halbestern der Kohlensäure, wie z.B. tert.-Niederalkoxycarbonyl oder gegebenenfalls substituierte Diphenylmethoxycarbonylreste, ferner auch ein tert.-Niederalkylrest; solche Reste können z.B. durch Behandeln mit geeigneten starken organischen Carbonsäuren, wie gegebenenfalls durch Halogen, insbesondere Fluor, substituierten Niederalkancarbonsäuren, in erster Linie mit Trifluoressigsäure (wenn notwendig, in Gegenwart eines aktivierenden Mittels, wie Anisol), sowie mit Ameisensäure abgespalten werden.

Unter reduktiv abspaltbaren Resten $X_1$ und/oder an funktionellen Gruppen, etwa den genannten Hydroxy- und/oder Aminogruppen, stehende Schutzgruppen in einem Rest R und/oder $Ar_1$ werden auch solche Gruppen verstanden, die beim Behandeln mit einem chemischen Reduktionsmittel (insbesondere mit einem reduzierenden Metall oder einer reduzierenden Metallverbindung) abgespalten werden. Solche Reste sind insbesondere 2-Halogenniederalkoxycarbonyl oder Arylmethoxycarbonyl, die z.B. beim Behandeln mit einem reduzierenden Schwermetall, wie Zink, oder mit einem reduzierenden Schwermetallsalz, wie einem Chrom(II)salz, z.B. -chlorid oder -acetat, üblicherweise in Gegenwart einer organischen Carbonsäure, wie Ameisensäure oder Essigsäure, und von Wasser abgespalten werden können.

Schutzgruppen, welche an in einem Rest R und/oder $Ar_1$ vorhandenen funktionellen Hydroxy- und/oder Aminogruppen stehen, entsprechen den vorstehend genannten und mittels der beschriebenen Methoden abspaltbaren und durch Wasserstoff ersetzbaren Gruppen, wobei solche Gruppen im Zuge des beschriebenen Verfahren gleichzeitig mit anderen Gruppen oder nachfolgend in einer getrennten Verfahrensmassnahme abgespalten werden.

Die obigen Reaktionen werden üblicherweise in Gegenwart eines Lösungsmittels oder Lösungsmittelgemisches durchgeführt, wobei geeignete Reaktionsteilnehmer gleichzeitig auch als solche fungieren

können, und, wenn notwendig, unter Kühlen oder Erwärmen, z.B. in einem offenen oder geschlossenen Gefäss und/oder in der Atmosphäre eines Inertgases, z.B. Stickstoff.

Ausgangstoffe der Formel X können auf an sich bekannte Weise hergestellt werden, z.B. analog den unter a) bis f) beschriebenen Verfahren. So kann man z.B. analog den in den Stufen aa) bis ah) beschriebenen Reaktionsschritten verfahren, wenn man anstelle der Verbindungen der Formeln XII, XIV, XVI, XVII oder XVIII solche Verbindungen einsetzt, die anstelle der oben definierten Gruppe Va die Gruppe der Formel

$$-COX-Alk_1-\underset{}{\overset{X_1}{\overset{|}{N}}}-Z-Alk_2-Y'-Ar_1 \qquad (Xa)$$

enthalten. Ein die Gruppe Xa enthaltender der Formel XII analog entsprechender Ausgangsstoff kann analog den dort beschriebenen Verfahrensmassnahmen erhalten werden.

Ausgangsstoffe der Formel X können ausserdem durch Umsetzung von Verbindungen der oben definierten Formel VI mit der Formel XIIe analog strukturierten Stoffen der Formel

$$H\overset{X_1}{\overset{|}{N}}-Z-Alk_2-Y'-Ar_1 \qquad (Xb)$$

erhalten werden, wobei die Herstellung von Ausgangsstoffen der Formel Xb wiederum analog der für die Umsetzung von Verbindungen XIIf mit solchen der Formel XIIg beschriebenen Verfahrensweise erfolgen kann.

Diese Umsetzungen werden in an sich bekannter Weise durchgeführt, üblicherweise in Anwesenheit von Lösungs- oder Verdünnungsmitteln bei erhöhter oder erniedrigter Temperatur, z.B. im Temperaturbereich

von etwa -15°C bis etwa 150°C unter normalem Druck oder im geschlossenen Gefäss gegebenenfalls unter Druck und/oder unter einem Schutzgas, etwa Stickstoff.

Verfahrensgemäss erhältliche Verbindungen der Formel I können in an sich bekannter Weise in andere Verbindungen der Formel I umgewandelt werden, etwa durch Umwandlung von in Verbindungen der Formel I enthaltenen Substituenten in andere von der Formel I umfasste Substituenten.

So kann man z.B. eine veresterte Carboxygruppe $-COOR_1$ durch Umesterung in eine andere Estergruppe überführen. Dabei verwendet man vorzugsweise entsprechende Alkoholverbindungen, die einen Siedepunkt aufweisen, der deutlich über demjenigen des Alkohols der veresterten Gruppe in der umzuwandelnden Verbindung der Formel I liegt, und führt die Reaktion z.B. in einem Ueberschuss der Hydroxyverbindung und/oder einem inerten organischen, vorzugsweise ebenfalls deutlich über dem Alkohol der veresterten Gruppe siedenden Lösungsmittel, vorzugsweise in Gegenwart eines Katalysators, z.B. eines Alkalimetall-niederalkanolats, wie Natrium- oder Kalium-methanolat oder -äthanolat, in der Wärme und üblicherweise unter Abdestillieren des freigesetzten Alkohols durch.

Verbindungen der Formel I, worin $R_4$ für Benzyl steht, können durch Abspalten der Benzylgruppe und deren Ersatz durch Wasserstoff in solche Verbindungen der Formel I umgewandelt werden, worin $R_4$ Wasserstoff ist. Für solche Abspaltungen eignet sich die katalytische Debenzylierung mittels Wasserstoff in Gegenwart eines Hydrierkatalysators, etwa Platin oder Raney-Nickel. Andererseits kann man Verbindungen der Formel I, worin $R_4$ Wasserstoff ist, in solche Verbindungen der Formel I umwandeln, worin $R_4$ eine Phenylniederalkylgruppe ist, z.B. durch Umsetzung mit einem Phenylniederalkanol, dessen Hydroxygruppe in reaktionsfähiger veresterter Form, z.B. als Halogenid wie als Chlorid oder Bromid, vorliegen kann. Zweckmässiger werden solche Umsetzungen in Gegenwart basischer Mittel, z.B. eines Oxids oder Hydroxids oder Carbonats eines Alkali-oder Erdalkalimetalls, wie Natriumhydroxid, Calciumhydroxid oder -carbonat und üblicherweise in Gegenwart eines Lösungsmittels, wie eines Niederalkanols, etwa Aethanol vorgenommen.

Verbindungen der Formel I, worin R einen der N-Oxidation zugänglichen azaheterocyclischen Rest, z.B. Pyridyl, darstellt, können mittels N-Oxidation in solche Verbindungen der Formel I umgewandelt werden, worin R einen N-oxidierten Azaheterocyclus, z.B. N-Oxido-pyridyl, bedeutet.

Die Oxidation kann in an sich bekannter Weise durchgeführt werden, z.B. durch Behandeln mit organischen Persäuren, wie Niederalkanpersäuren oder Arenpersäuren, wie gegebenenfalls geeignet substituierten Perbenzoesäuren, z.B. Peressig- oder 3-Chlorperbenzoesäure, vorzugsweise bei Zimmertemperatur oder etwas darüber liegender Reaktionstemperatur, oder mit wässrigem Wasserstoffperoxid, z.B. bei Temperaturen von bis zu 100°C, in Gegenwart oder Abwesenheit von Niederalkansäuren, z.B. Essigsäure. Dabei muss, insbesondere bei Verwendung von Persäuren, darauf geachtet werden, dass keine Ueberoxidation aufgrund einer zu langen Reaktionsdauer eintreten kann.

Je nach Reaktionsbedingungen können Verbindungen der Formel I freier Form oder in Form von Salzen erhalten werden.

So können erhaltene Säureadditionssalze in an sich bekannter Weise, z.B. durch Behandeln mit einer Base, wie einem Alkalimetallhydroxid, in die freien Verbindungen oder z.B. durch Behandeln mit geeigneten Säuren oder Derivaten davon in andere Salze umgewandelt werden. Erhaltene freie Verbindungen der Formel I können, z.B. durch Behandeln mit Säuren oder entsprechenden Anionenaustauschern, in ihre Salze umgewandelt werden.

Infolge der engen Beziehung zwischen den Verbindungen der Formel I in freier Form und in Form von Salzen, sind im Vorausgegangenen und nachfolgend unter den freien Verbindungen oder ihren Salzen sinn-und zweckgemäss gegebenenfalls auch die entsprechenden Salze bzw. freien Verbindungen zu verstehen.

Die Verbindungen der Formel I, einschliesslich deren Salze, können auch in Form ihrer Hydrate erhalten werden, oder ihre Kristalle können z.B. das zur Kristallisation verwendete Lösungsmittel einschliessen.

Die Verbindungen der Formel I können, je nach Verfahrensreaktion und/oder Art der Ausgangsstoffe, in Form von Racematgemischen, Racematen oder optischen Antipoden erhalten werden.

Erhaltene Racematgemische können auf Grund der chemischen Struktur und der physikalisch-chemischen Unterschiede der Racemate in bekannter Weise in die reinen Racemate bzw. Diastereomeren aufgetrennt werden, beispielsweise durch Chromatographie und/oder fraktionierte Kristallisation.

Racemate lassen sich nach an sich bekannten Methoden in die optischen Antipoden zerlegen, beispielsweise durch Umkristallisation aus einem optisch aktiven Lösungsmittel, mit Hilfe von geeigneten Mikroorganismen oder durch Umsetzen einer Verbindung der Formel I mit salzbildenden, z.B. basischen, Eigenschaften mit einem optisch aktiven, salzbildenden Mittel, wie einer optisch aktiven Säure, und Trennen der auf diese Weise erhaltenen Gemische von Salzen, z.B. auf Grund ihrer verschiedenen Löslichkeiten, in die diastereomeren Salze, aus denen die Antipoden, z.B. durch Behandeln mit einer Base, freigesetzt werden können.

Optische Antipoden von neutralen Verbindungen der Formel I kann man z.B. auch gemäss Verfahren c) unter Verwendung einer optisch aktiven Säure der Formel V ($Ac^o$ bedeutet Carboxy, $Ac_1^o$ ist verestertes oder amidiertes Carboxy) erhalten, wobei man diese z.B. aus der entsprechenden racemischen Säure in üblicher Weise, z.B. durch Salzbildung mit einer optisch aktiven Base, Trennung der diastereomeren Salze und Freisetzung der optisch aktiven Säure und deren Umwandlung in eine der Formel I entsprechende, die Gruppe $-COOR_1-$ enthaltende Verbindung erhält.

Ferner kann man z.B. Verbindungen der Formel I mit der Gruppe $-COOR_1-$ unter Verwendung eines optisch aktiven Alkohols nach dem oben beschriebenen Verfahren umestern und das so erhältliche Diastereomerengemisch, z.B. mittels fraktionierter Kristallisation, in die Antipoden auftrennen.

Vorteilhafterweise isoliert man aus einem Diastereomerengemisch bzw. Racemat das pharmakologisch aktivere Diastereomere bzw. den aktiveren Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivats, z.B. Salzes, und/oder seiner Racemate bzw. Antipoden verwendet oder unter den Reaktionsbedingungen bildet.

Bei den Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Zwischenprodukte sowie Verfahren zur deren Herstellung bilden ebenfalls einen Gegenstand der vorliegenden Erfindung.

Die Erfindung betrifft ebenfalls die Verwendung der Verbindungen der Formel I oder von pharmazeutisch verwendbaren Salzen von solchen Verbindungen mit salzbildenden Eigenschaften, insbesondere als

pharmakologisch wirksame Verbindungen, in erster Linie als Coronardilatatoren und Antihypertensiva zur Behandlung von cardiovasculären Krankheitszuständen, wie Angina pectoris und ihre Folgen, Gefäss-Spasmen, zentrale und periphere Durchblutungsstörungen, hoher Blutdruck, Arrhythmien und Herzinsuffizienz sowie von Migräne, ferner zur Hemmung der Plättchenaggregation. Dabei kann man sie, vorzugsweise in Form von pharmazeutischen Präparaten, in einem Verfahren zur prophylaktischen und/oder therapeutischen Behandlung des tierischen und menschlichen Körpers, insbesondere zur Behandlung von cardiovasculären Krankheitszuständen, wie Angina pectoris und ihre Folgen, Gefäss-Spasmen, hoher Blutdruck und Herzinsuffizienz verwenden.

Die Dosierung des Wirkstoffs, der allein oder zusammen mit dem üblichen Träger- und Hilfsmaterial verabreicht wird, hängt von der zu behandelnden Spezies, deren Alter und individuellem Zustand, sowie der Verabreichungsweise ab. Die täglichen Dosen liegen z.B. für Mammalien mit einem Körpergewicht von etwa 70 kg, je nach Art der Erkrankung, individuellem Zustand und Alter, vorzugsweise zwischen etwa 10 und 500 mg, insbesondere zwischen etwa 20 mg und etwa 200 mg, und speziell etwa bei 70 mg bis 150 mg bei oraler Verabreichung.

Die Erfindung betrifft im weiteren pharmazeutische Präparate, die Verbindungen der Formel I oder pharmazeutisch verwendbare Salze von solchen Verbindungen mit salzbildenden Eigenschaften als Wirkstoffe enthalten, Verfahren zu ihrer Herstellung sowie die Verwendung von Verbindungen der Formel I oder deren pharmazeutisch verwendbaren Salzen zur Herstellung von Arzneimitteln als Coronardilatatoren und Antihypertensiva zur Behandlung von cardiovasculären Krankheitszuständen, wie Angina pectoris und ihre Folgen, Gefäss-Spasmen, zentrale und periphere Durchblutungsstörungen, hoher Blutdruck, Arrhythmien und Herzinsuffizienz, sowie von Migräne, ferner zur Anwendung als Hemmer bei Plättchenaggregation.

Bei den erfindungsgemässen pharmazeutischen Präparaten handelt es sich um solche zur enteralen, wie peroralen oder rektalen, weiter zur sublingualen, sowie zur parenteralen Verabreichung an Warmblüter. Entsprechende Dosiseinheitsformen, insbesondere zur peroralen und/oder sublingualen Verabreichung, z.B. Dragées, Tabletten oder Kapseln, enthalten vorzugsweise von etwa 10 mg bis etwa 300 mg, insbesondere von etwa 20 mg bis etwa 200 mg einer Verbindung der Formel I oder eines pharmazeutisch annehmbaren Salzes einer zur Salzbildung befähigten entsprechenden Verbindung zusammen mit pharmazeutisch verwendbaren Trägerstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder, wenn erwünscht, Sprengmittel, wie die genannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyäthylenglykol und/oder Titandioxid enthalten, oder Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können. Bevorzugt sind u.a. Kapseln, die sowohl leicht zerbissen werden können, um durch sublinguale Aufnahme des Wirkstoffes eine möglichst rasche Wirkung zu erzielen, als auch unzerkaut geschluckt werden können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat, oder Triglyceride verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die pharmazeutischen Präparate der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die nachfolgenden Beispiele illustrieren die vorstehend beschriebene Erfindung, ohne jedoch ihren Umfang in irgendeiner Weise einschränken zu sollen. Temperaturen werden in Celsiusgraden angegeben.

Beispiel 1: Eine Lösung von 6,2 g 2,6-Dimethyl-4-(3-nitrophenyl)-1,4-dihydropyridin-3,5-dicarbonsäure-3-methylester-5-[2-[N-(4-äthylendioxy-4-(4-fluorphenyl)-n-butyl)-N-methyl-amino]äthylester] in 100 ml 2N-Salzsäure und 50 ml Aethanol wird während 5 Stunden unter Rückfluss erhitzt und die Reaktionslösung anschliessend im Vakuum eingeengt. Den Rückstand versetzt man mit Natronlauge bis zur stark alkalischen Reaktion und extrahiert das Reaktionsgemisch mit Methylenchlorid. Nach dem Trocknen der organischen Phase über Natriumsulfat wird zur Trockne abgedampft und der Rückstand über der 300-fachen Menge Kieselgel chromatographiert, wonach man den 2,6-Dimethyl-4-(3-nitrophenyl)-1,4-dihydropyridin-3,5-dicarbonsäure-3-methylester-5-[2-[N-(4-(4-fluorphenyl)-4-oxo-n-butyl)-N-methyl-amino]äthylester] der folgenden Formel

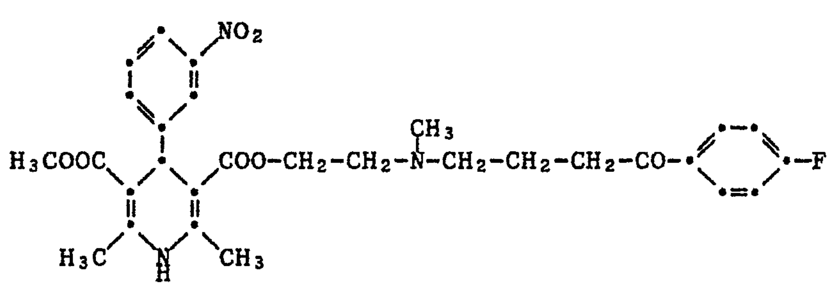

erhält. Er weist folgende Kenndaten auf:

250 MHz FT-$^1$H-NMR ($CDCl_3$): 1.88 (m, 2H, $-C-CH_2-C-$); 2.24 (s, 3H, $NCH_3$); 2.36 (s, 6H, Dihydropyridyl-$CH_3$); 2.44, 2.60 (2t, 4H, N-$CH_2$); 2.96 (t, 2H, $-CH_2CO$) 3.64 (s, 3H, $COOCH_3$); 4.12 (m, 2H, $-OCH_2-$); 5.12 (s, 1H, 4-Dihydropyridyl-H); 5.8 (s, 1H, NH); 7.1 - 8.15 (m, 8H, Phenyl-H).

Der Ausgangsstoff kann wie folgt hergestellt werden:
Ein Gemisch von 4 g 2,6-Dimethyl-4-(3-nitrophenyl)-1,4-dihydropyridin-3,5-dicarbonsäure-3-methylester-5-(2-chloräthylester) (DOS 2,407,115), 2,42 g 2-(4-Fluorphenyl)-2-[3-methylamino-propyl-(1)]-1,3-dioxolan und 1,44 g Hünigbase wird in einem auf 140 - 150°C vorgeheizten Oelbad während 90 Minuten unter Rühren erhitzt. Nach dem Abkühlen wird die erstarrte Schmelze in Essigsäureäthylester aufgenommen, die organische Phase mehrmals mit gesättigter Kochsalzlösung, dann mit Wasser gewaschen, über Natriumsulfat getrocknet und eingedampft, wonach man den 2,6-Dimethyl-4-(3-nitrophenyl)-1,4-dihydropyridin-3,5-dicarbonsäure-3-methylester-5-[2-[N-(4-äthylendioxy-4-(4-fluorphenyl)-n-butyl)-N-methyl-amino]äthylester] als Rohprodukt erhält, welcher als solches weiterverarbeitet wird.

Beispiel 2: Analog den in der Beschreibung beschriebenen und dem im vorstehenden Beispiel illustrierten Verfahren können, ausgehend von entsprechenden Ausgangsstoffen, oder, sofern sie salzbildende Eigenschaften haben, auch deren Salzen, auch folgende Verbindungen der Formel I oder, sofern diese salzbildende Eigenschaften haben, auch deren Salze, insbesondere deren pharmazeutisch verwendbaren, nicht-toxischen Säureadditionssalze hergestellt werden:

a) 2,6-Dimethyl-4-(3-nitrophenyl)-1,4-dihydropyridin-3,5-dicarbonsäure-3-methylester-5-[2-[N-(4-(4-fluorphenyl)-1,4-dioxo-n-butyl)-N-methyl-amino]äthylester].

b) 2,6-Dimethyl-4-(3-nitrophenyl)-1,4-dihydropyridin-3,5-dicarbonsäure-3-methylester-5-[2-[N-äthyl-N-(4-(4-fluorphenyl)-4-oxo-n-butyl)amino]äthylester].

c) 2,6-Dimethyl-4-(3-nitrophenyl)-1,4-dihydropyridin-3,5-dicarbonsäure-3-methylester-5-[2-[N-benzyl-N-(4-(4-fluorphenyl)-4-oxo-n-butyl)amino]äthylester].

d) 2,6-Dimethyl-4-(3-nitrophenyl)-1,4-dihydropyridin-3,5-dicarbonsäure-3-methylester-5-[2-[N-methyl-N-(4-phenyl-1,4-dioxo-n-butyl)amino]äthylester].

e) 2,6-Dimethyl-4-(3-nitrophenyl)-1,4-dihydropyridin-3,5-dicarbonsäure-3-methylester-5-[2-[N-methyl-N-(4-phenyl-4-oxo-n-butyl)amino]-äthylester].

f) 2,6-Dimethyl-4-(3-nitrophenyl)-1,4-dihydropyridin-3,5-dicarbonsäure-3-methylester-5-[2-[N-benzyl-N-(4-phenyl-4-oxo-n-butyl)amino]-äthylester].

g) 2,6-Dimethyl-4-(3-nitrophenyl)-1,4-dihydropyridin-3,5-dicarbonsäure-3-methylester-5-[2-[N-methyl-N-(3-(2-thenoyl)propyl)amino]-äthylester].

h) 2,6-Dimethyl-4-(3-nitrophenyl)-1,4-dihydropyridin-3,5-dicarbonsäure-3-methylester-5-[2-[N-benzyl-N-(3-(2-thenoyl)propyl)amino]-äthylester].

Beispiel 3: 0,778 g 2,6-Dimethyl-4-(3-nitrophenyl)-1,4-dihydropyridin-3,5-dicarbonsäure-3-methylester-5-(2-methylamino)äthylester (Chem. Pharm. Bull. 28, 2605, 1980), 0,401 g ω-Chlor-4-fluorobutyrophenon, 0,414 g Kaliumcarbonat und 0,3 g (0.002 Mol) Natriumiodid werden in 20 ml Isopropanol während 60 h unter Rückfluss erhitzt. Anschliessend dampft man im Vakuum ein, versetzt den Rückstand mit Natronlauge bis zur alkalischen Reaktion, extrahiert das Reaktionsgemisch mit Methylenchlorid und wäscht die Methylenchloridlösung mit wässriger Natriumthiosulfatlösung nach. Nach dem Trocknen der organischen Phase über Natriumsulfat wird zur Trockne eingedampft. Den Rückstand chromatographiert man über der 300-fachen

Menge Kieselgel. Man erhält so den 2,6-Dimethyl-4-(3-nitrophenyl)-1,4-dihydropyridin-3,5-dicarbonsäure-3-methylester-5-[2-[N-(4-(4-fluorphenyl)-4-oxo-n-butyl)-N-methyl-amino]äthylester], Charakterisierung siehe Beispiel 1.

Beispiel 4: Zu einem Gemisch von 47 ml Dimethylformamid und 44 ml Acetonitril wird unter Rühren bei -30°C während 20 Minuten ein Gemisch von 3,55 g Oxalylchlorid und 25 ml Acetonitril getropft. Die weisse Suspension wird während 30 Minuten bei -30°C gerührt, dann werden 13,5 g 2,6-Dimethyl-4-(3-nitrophenyl)-1,4-dihydropyridin-3,5-dicarbonsäure-3-methylester zugegeben. Es wird weiter gerührt, bis eine gelbe Lösung entsteht. Bei -30°C wird hierauf eine Lösung von 11,7 g eines Gemisches von N-4-(4-Fluorphenyl)-4-oxo-n-butyl-N-methyl-2-amino-1-phenyl-äthanol und N-4-(4-Fluorphenyl)-4-oxo-n-butyl-N-methyl-2-amino-2-phenyl-äthanol in 25 ml Pyridin zugetropft. Es wird über Nacht bei Raumtemperatur ausgerührt und dann unter vermindertem Druck eingedampft. Der Rückstand wird in Methylenchlorid gelöst und mit Wasser und Soda extrahiert. Die organische Phase wird über Natriumsulfat getrocknet, eingeengt, und der Rückstand wird in einem Gemisch von Methylenchlorid/Methanol 98:2 mehrmals chromatographiert. Man erhält so den 2,6-Dimethyl-4-(3-nitrophenyl)-1,4-dihydropyridin-3,5-dicarbonsäure-3-methylester-5-[2-[N-(4-(4-fluorphenyl)-4-oxo-n-butyl)-N-methyl-amino]-2-phenyl-äthylester] in amorpher Form.

$^{13}C$-NMR ($CDCl_3$) (nur die aliphatischen Kohlenstoffatome der Seitenkette sind angegeben): 66.7, 66.6 (NCH); 64.2, 64.0 ($OCH_2$); 53.5, 53.4 ($NCH_2$); 51.11, 51.08 ($OCH_3$); 38.4 ($NCH_3$); 35.8 ($\underline{C}H_2CO$); 21.7 ($\underline{C}H_2CH_2CO$); $R_f$: 0.41 (Methylenchlorid/Methanol: 95/5). Aus dem gleichen Ansatz kann der 2,6-Dimethyl-4-(3-nitrophenyl)-1,4-dihydropyridin-3,5-dicarbonsäure-3-methylester-5-[2-[N-(4-(4-fluorphenyl)-4-oxo-n-butyl)-N-methyl-amino]-1-phenyl-äthylester] in amorpher Form isoliert werden.

$^{13}C$-NMR ($CDCl_3$) (nur die aliphatischen Kohlenstoffatome der Seitenkette sind angegeben): 73.3 (d, OCH); 62.7 (t, $N\underline{C}H_2CH$); 56.6 (t, $N\underline{C}H_2CH_2$); 42.5 (q, $NCH_3$); 35.9 (t, $\underline{C}H_2CO$); 21.5 (t, $\underline{C}H_2CH_2CO$); $R_f$: 0,38 (Methylenchlorid/Methanol: 95/5).

Die Ausgangsmaterialien können wie folgt hergestellt werden: Der 2,6-Dimethyl-4-(3-nitrophenyl)-1,4-dihydropyridin-3,5-dicarbonsäure-3-methylester ist in der EP-A-11706 (Bayer) beschrieben.

Zur Herstellung der Aminoalkohole wird ein Gemisch von 20 g 2-(3-Chlorpropyl)-2-(4-fluorphenyl)-1,3-dioxolan, 9,9 g N-Benzylmethylamin und 22,5 g Kaliumcarbonat in 50 ml Aceton während 3 Tagen unter Rückfluss erhitzt. Dann wird abfiltriert, eingedampft und der Rückstand durch Blitzchromatographie unter Verwendung eines Gemisches von Methylenchorid/Methanol (98:2) gereinigt. Das erhaltene Aminodioxolan wird in Alkohol mit Pd/C bei Raumtemperatur debenzyliert.

Ein Gemisch von 4,8 g des so erhaltenen 2-(3-Methylaminopropyl)-2-(4-fluorphenyl)-1,3-dioxolan mit 2,9 g Phenyläthylenoxid wird in 50 ml Isopropanol 15 Stunden unter Rückfluss erhitzt. Nach dem Eindampfen wird der Rückstand durch Blitzchromatographie unter Verwendung eines Gemisches von Methylenchlorid/Methanol (98:2) gereinigt. Das Gemisch der so entstandenen ketalisierten Aminoalkohole wird direkt weiter verarbeitet.

6.0 g des ketalisierten Aminoalkoholgemisches werden mit 100 ml 2N HCl und 150 ml Aceton 4 Stunden unter Rückfluss erhitzt. Die Lösung wird eingedampft, und der Rückstand wird zwischen Methylenchlorid und Wasser verteilt. Die wässrige Lösung wird mit Natriumbicarbonat alkalisch gestellt und mit Methylenchlorid extrahiert. Die organische Phase wird getrocknet und eingedampft. Das Gemisch aus N-4-(4-Fluorphenyl)-4-oxo-n-butyl-N-methyl-2-amino-1-phenyläthanol und N-4-(4-Fluorphenyl)-4-oxo-n-butyl-N-methyl-2-amino-2-phenyläthanol wird direkt mit der Dihydropyridincarbonsäure verestert.

Beispiel 5: Tabletten enthaltend 20 mg an aktiver Substanz werden in folgender Zusammensetzung in üblicher Weise hergestellt:

Zusammensetzung:

| | |
|---|---|
| 2,6-Dimethyl-4-(3-nitrophenyl)-1,4-dihydropyridin-3,5-dicarbonsäure-3-methylester-5-[2-[N-(4-(4-fluorphenyl)-4-oxo-n-butyl)-N-methyl-amino]äthylester] | 20 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung:

Der Wirkstoff wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist. Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt, und die Mischung wird zu Tabletten von 145 mg Gewicht mit Bruchkerbe verpresst.

Beispiel 6: Kapseln enthaltend 10 mg an aktiver Substanz werden wie folgt auf übliche Weise hergestellt:

Zusammensetzung:

| | |
|---|---|
| 2,6-Dimethyl-4-(3-nitrophenyl)-1,4-dihydropyridin-3,5-dicarbonsäure-3-methylester-5-[2-[N-(4-(4-fluorphenyl)-4-oxo-n-butyl)-N-methyl-amino]äthylester] | 2500 mg |
| Talkum | 200 mg |
| Kolloidale Kieselsäure | 50 mg |

Herstellung:

Die aktive Substanz wird mit Talkum und kolloidaler Kieselsäure innig gemischt, das Gemisch durch ein Sieb mit 0,5 mm Maschenweite getrieben und dieses in Portionen von jeweils 11 mg in Hartgelatinekapseln geeigneter Grösse abgefüllt.

Beispiel 7: Eine Lösung von 5,0 g 2,6-Dimethyl-4-(3-nitrophenyl)-1,4-dihydropyridin-3,5-dicarbonsäure-3-methylester-5-[2-[N-(4-(4-fluorphenyl)-4-oxo-n-butyl)-N-methylamino]äthylester] in 9 ml 1-N Salzsäure wird mit Wasser auf 5000 ml aufgefüllt. Die sterile Lösung wird in Ampullen zu 5 ml abgefüllt, die in 5 ml Lösung 5 mg Wirkstoff enthalten.

Beispiel 8: Anstelle der in den Beispielen 5 bis 7 als aktive Substanz verwendeten Verbindungen können auch die in den Beispielen 2 und 4 genannten Verbindungen der Formel I oder, sofern sie salzbildende Eigenschaften besitzen, deren pharmazeutisch annehmbare, nicht-toxische Säureadditionssalze als Wirkstoffe in Tabletten, Dragées, Kapseln etc. verwendet werden.

0214094

Patentansprüche

1. Verbindungen der Formel

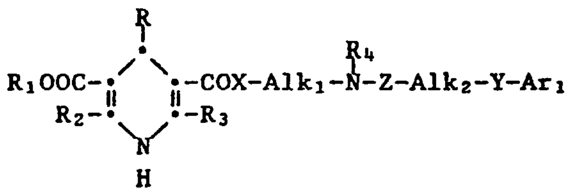

(I),

worin R einen carbocyclischen oder heterocyclischen Arylrest bedeutet, $R_1$ Niederalkyl darstellt, eine der Gruppen $R_2$ und $R_3$ für Niederalkyl und die andere für Niederalkyl, Cyan oder Amino steht, X Sauerstoff oder die Gruppe -NH- darstellt, $Alk_1$ Niederalkylen oder gegebenenfalls substituiertes Phenylniederalkylen bedeutet, das die Gruppe X vom Stickstoffatom durch mindestens zwei Kohlenstoffatome trennt, $R_4$ Wasserstoff, Niederalkyl oder gegebenenfalls substituiertes Phenylniederalkyl bedeutet, Z für die Gruppe -(C=O)- oder eine einfache Bindung steht, $Alk_2$ Niederalkylen darstellt, welches die Gruppen Z und Y vorzugsweise durch 2 - 4 Kohlenstoffatome trennt, Y für die Gruppe -(C=O)- steht, und $Ar_1$ einen monocyclischen carbocyclischen oder heterocyclischen Arylrest darstellt, sowie Salze solcher Verbindungen mit salzbildenden Eigenschaften.

2. Verbindungen der Formel I gemäss Anspruch 1, in welchen R für einen mono- oder bicyclischen carbocyclischen Arylrest oder für einen fünf- oder sechsgliedrigen, ein bis vier Ringstickstoffatome, ein Ringsauerstoff- oder Ringschwefelatom, oder ein oder zwei Ringstickstoffatome zusammen mit einem Ringsauerstoff- oder einem Ringschwefelatom als Ringglieder enthaltenden, monocyclischen Heteroarylrest steht, der über ein Ringkohlenstoffatom mit dem Kohlenstoffatom der 4-Stellung des 1,4-Dihydropyridinrings verbunden ist, und der gegebenenfalls einen ankondensierten Benzoring enthält, und insbesondere Phenyl, Naphthyl, Pyrryl, Pyrazolyl, Imidazolyl, Triazolyl, Tetrazolyl, Furyl, Thienyl, Isoxazolyl, Oxazolyl,

Oxadiazolyl, Isothiazolyl, Thiazolyl, Thiadiazolyl, Pyridyl, Pyridazinyl, Pyrimidinyl, Pyrazinyl, Triazinyl, Indolyl, Isoindolyl, Benzimidazolyl, Benzoxadiazolyl, Benzofuranyl, Benzofurazanyl, Benzothienyl, Benzthiazolyl, 2,1,3-Benzthiadiazolyl, Chinolinyl oder Isochinolinyl bedeutet, wobei in diesen Resten Ringkohlenstoffatome gegebenenfalls durch Niederalkyl, Niederalkenyl, Niederalkinyl, Niederalkylen, Cycloalkyl, Phenyl und/oder Phenylniederalkyl, wobei Niederalkyl, Phenyl oder Phenylniederalkyl gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, und cyclische Reste auch Niederalkyl, das seinerseits wie angegeben substituiert sein kann, als Substituenten enthalten können, und/oder durch Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Nitro, Amino, N-Niederalkyl-amino, N,N-Diniederalkylamino, N-Niederalkyl-N-phenylniederalkyl-amino, Niederalkylenamino, Oxaniederalkylenamino, Thianiederalkylenamino und/oder Azaniederalkylenamino, worin das Azastickstoffatom durch Niederalkyl, Phenyl oder Phenylniederalkyl substituiert sein kann, wobei diese Reste Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, die cyclischen Reste auch Niederalkyl als Substituenten enthalten können, und/oder durch Niederalkanoylamino, Azido, Niederalkanoyl, Carboxy, Niederalkoxycarbonyl, Carbamoyl, N-Niederalkyl-carbamoyl, N,N-Diniederalkylcarbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl, und/oder Ringstickstoffatome gegebenenfalls durch Niederalkyl, das als Substituenten gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl oder Cyan enthalten kann, oder durch Hydroxy oder Oxido substituiert sein können, $R_1$ Niederalkyl ist, einer der Reste $R_2$ und $R_3$ für Niederalkyl und der andere für Niederalkyl, Cyan oder Amino steht, X Sauerstoff oder die Gruppe -NH- bedeutet, $Alk_1$ Niederalkylen oder gegebenenfalls insbesondere im Phenylteil durch gegebenenfalls veräthertes oder verestertes Hydroxy, durch gegebenenfalls veräthertes oder verestertes Hydroxy substituiertes Niederalkyl oder Niederalkoxy oder durch Carboxy, Niederalkoxycarbonyl oder Cyan substituiertes Phenylniederalkylen darstellt, das die Gruppe X vom Stickstoffatom durch 2 bis 8 Kohlenstoffatome trennt, $R_4$ Wasserstoff, Niederalkyl, gegebenenfalls insbesondere im Phenylteil durch gegebenenfalls veräthertes oder verestertes Hydroxy, durch gegebenenfalls veräthertes oder verestertes Hydroxy substituiertes Niederalkyl oder Niederalkoxy substituiertes Phenylniederalkyl bedeutet, Z die Gruppe -(C=O)- oder eine einfache Bindung darstellt, $Alk_2$ Niederalkylen bedeutet, welches die Gruppen Z und Y durch 2 bis 6 Kohlenstoffatome trennt, Y für die Gruppe -(C=O)-steht, und $Ar_1$ einen monocyclischen carbocyclischen Aryl- oder Heteroarylrest darstellt, und insbesondere Phenyl, Naphthyl, Pyrryl, Furyl, Thienyl oder Pyridyl bedeutet, wobei diese Reste wie vorstehend im Zusammenhang mit R definiert substituiert sein können, sowie Salze solcher Verbindungen mit salzbildenden Eigenschaften.

3. Verbindungen der Formel I gemäss Anspruch 1, worin R und $Ar_1$ jeweils für Phenyl oder Naphthyl stehen, das gegebenenfalls durch Niederalkyl, Phenyl und/oder Phenylniederalkyl, wobei solche Reste ihrerseits Hydroxy, Niederalkoxy, Halogenniederalkoxy, Niederalkylendioxy, Halogen, Carboxy, Niederalkoxycarbonyl und/oder Cyan, die cyclischen Reste auch Niederalkyl als Substituenten enthalten können, und/oder durch Hydroxy, Niederalkoxy, Halogenniederalkoxy, Niederalkenyloxy, Halogenniederalkenyloxy, Niederalkylendioxy, Halogen, Nitro, Amino, N-Niederalkylamino, N,N-Diniederalkylamino, Niederalkanoylamino, Carboxy, Niederalkoxycarbonyl, Carbamoyl, Cyan,

Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl substituiert ist, oder R und $Ar_1$ jeweils für über ein Ringkohlenstoffatom gebundenes Pyrryl, Furyl, Thienyl, Pyridyl, 1-Oxido-pyridyl oder Imidazolyl, R auch für Benzofurazanyl oder Benzoxadiazolyl steht, wobei solche Reste gegebenenfalls, wie für einen Phenyl- oder Naphthylrest R bzw. $Ar_1$ angegeben, substituiert sind, und insbesondere Niederalkyl, Niederalkoxy, Halogen und/oder gegebenenfalls durch Niederalkyl, Niederalkoxy, Halogen und/oder Nitro substituiertes Phenyl als Substituenten enthalten können, $R_1$ Niederalkyl ist, $R_2$ und $R_3$ jeweils Niederalkyl bedeuten, oder eine der Gruppen $R_2$ und $R_3$ Niederalkyl ist und die andere Niederalkyl oder Cyan darstellt, X Sauerstoff oder die Gruppe -NH- bedeutet, $Alk_1$ für die Gruppe $-(CH_2)_n-$ steht, worin n Werte von 2 bis 6 darstellt, $R_4$ Wasserstoff, Niederalkyl oder Phenylniederalkyl bedeutet, Z die Gruppe -(C=O)- oder eine einfache Bindung darstellt, $Alk_2$ für die Gruppe $-(CH_2)_m-$ steht, worin m Werte von 2 bis 6, vorzugsweise 2 bis 4 bedeutet, und Y für die Gruppe -(C=O)- steht, sowie Salze solcher Verbindungen mit salzbildenden Eigenschaften.

4. Verbindungen der Formel I gemäss Anspruch 1, worin R und $Ar_1$ jeweils für Phenyl stehen, das gegebenenfalls durch Niederalkyl, Hydroxy, Niederalkoxy, Halogenniederalkoxy, Halogenniederalkenyloxy, Niederalkylendioxy, Halogen, Trifluormethyl, Nitro, Niederalkanoylamino, gegebenenfalls durch gegebenenfalls verestertes oder veräthertes Hydroxy substituiertes Phenyl oder Phenylniederalkyl, und/oder Cyan substituiert ist, wobei ein Phenylrest R bzw. $Ar_1$ einen oder mehrere, gleiche oder verschiedene der genannten Substituenten aufweisen kann, oder R und $Ar_1$ jeweils Pyridyl, Furyl, 1-Oxido-pyridyl oder Thienyl, R auch Benzofurazanyl bedeuten, das durch Niederalkyl oder Halogen substituiert sein kann, $R_1$, $R_2$ und $R_3$ jeweils Niederalkyl bedeuten, X für Sauerstoff oder die Gruppe -NH- steht, $Alk_1$ und $Alk_2$ jeweils die Gruppe $-(CH_2)_n-$ darstellen, worin n Werte von 2 bis 4 bedeutet, $R_4$ Wasserstoff oder Niederalkyl bedeutet, Z die Gruppe -(C=O)- oder eine einfache Bindung darstellt,

und Y für die Gruppe -(C=O)- steht, sowie Salze solcher Verbindungen mit salzbildenden Eigenschaften.

5. Verbindungen der Formel I gemäss einem der Ansprüche 3 oder 4, worin $Alk_1$ die Gruppe $-(CH_2)_n-$ darstellt, worin n Werte von 2 bis 4 bedeutet und worin ein Wasserstoff durch Phenyl ersetzt ist, das gegebenenfalls durch Niederalkyl, Hydroxy, Niederalkoxy, Halogenniederalkoxy, Niederalkylendioxy, Halogen, Carboxy, Niederalkoxycarbonyl und/oder Cyan substituiert ist.

6. Verbindungen der Formel I gemäss Anspruch 1, worin R für unsubstituiertes oder vorzugsweise durch Niederalkyl, Niederalkoxy, Halogenniederalkoxy oder Halogenniederalkenyloxy, worin Halogen eine Atomnummer bis und mit 35 hat, Halogen mit einer Atomnummer bis und mit 35, Trifluormethyl, Nitro und/oder Cyan mono- oder di-substituiertes Phenyl steht, wobei Substituenten die 2- und/oder 3-Stellung einnehmen, $R_1$, $R_2$ und $R_3$ jeweils Niederalkyl bedeuten, X für Sauerstoff oder die Gruppe -NH- steht, $Alk_1$ und $Alk_2$ jeweils die Gruppe $-(CH_2)_n-$ darstellen, worin n Werte von 2 bis 4 bedeutet, $R_4$ Wasserstoff oder Niederalkyl darstellt, Z für eine einfache Bindung steht, Y für die Gruppe -(C=O)- steht, und $Ar_1$ gegebenenfalls durch Halogen mit einer Atomnummer bis und mit 35 substituiertes Phenyl oder Pyridyl bedeutet, sowie Salze solcher Verbindungen mit salzbildenden Eigenschaften.

7. Verbindungen der Formel I gemäss Anspruch 6, worin $Alk_1$ die Gruppe $-(CH_2)_n-$ darstellt, worin n Werte von 2 bis 4 bedeutet und worin ein Wasserstoff durch Phenyl ersetzt ist, das gegebenenfalls durch Niederalkyl, Niederalkoxy, Hydroxy oder Halogen substituiert ist.

8. Verbindungen der Formel I gemäss Anspruch 1, worin R für durch Halogen mit einer Atomnummer bis und mit 35 mono- oder disubstituiertes oder durch Trifluormethyl, Nitro oder Cyan monosubstituiertes Phenyl steht, wobei Substituenten die 2- und/oder 3-Stellung einnehmen, $R_1$, $R_2$ und $R_3$ jeweils Niederalkyl bedeuten, X für

Sauerstoff oder die Gruppe -NH- steht, $Alk_1$ und $Alk_2$ jeweils die Gruppe $-(CH_2)_n$-darstellen, worin n Werte von 2 bis 3 bedeutet, $R_4$ Wasserstoff oder Niederalkyl darstellt, Z eine einfache Bindung ist, Y für die Gruppe -(C=O)- steht, und $Ar_1$ gegebenenfalls durch Halogen mit einer Atomnummer bis und mit 35 substituiertes Phenyl ist, sowie Salze solcher Verbindungen mit salzbildenden Eigenschaften.

9. Verbindungen der Formel I gemäss Anspruch 8, worin $Alk_1$ die Gruppe $-(CH_2)_n-$ darstellt, worin n Werte von 2 bis 3 bedeutet und worin ein Wasserstoff durch Phenyl ersetzt ist.

10. Verbindungen der Formel I gemäss Anspruch 1, worin R für durch Halogen mit einer Atomnummer von bis und mit 35 mono- oder disubstituiertes oder durch Nitro oder Cyan monosubstituiertes Phenyl steht, wobei Substituenten die 2- und/oder 3-Stellung einnehmen, $R_1$, $R_2$ und $R_3$ jeweils Niederalkyl bedeuten, X für Sauerstoff steht, $Alk_1$ und $Alk_2$ jeweils die Gruppe $-(CH_2)_n-$ darstellen, worin n die Werte von 2 bis 3 bedeutet, $R_4$ Niederalkyl darstellt, Z eine einfache Bindung ist, Y für die Gruppe -(C=O)- steht und $Ar_1$ durch Halogen mit einer Atomnummer bis und mit 35 substituiertes Phenyl ist, sowie Salze solcher Verbindungen mit salzbildenden Eigenschaften.

11. Verbindungen der Formel I gemäss Anspruch 1, worin R für in 2-und/oder 3-Stellung durch Halogen mit einer Atomnummer von bis und mit 35 mono- oder disubstituiertes oder durch Nitro oder Cyan monosubstituiertes Phenyl steht, $R_1$, $R_2$ und $R_3$ Niederalkyl mit bis zu 4 Kohlenstoffatomen bedeuten, X für Sauerstoff steht, $Alk_1$ für unsubstituiertes oder durch Phenyl substituiertes $\alpha,\omega$-Alkylen mit 2 oder 3 Kohlenstoffatomen steht, $Alk_2$ für $\alpha,\omega$-Alkylen mit 2 oder 3 Kohlenstoffatomen steht, $R_4$ für unsubstituiertes oder durch Phenyl substituiertes Niederalkyl mit bis zu 4 Kohlenstoffatomen steht, Z eine einfache Bindung ist, Y für die Gruppe -(C=O)- steht und $Ar_1$ unsubstituiertes oder durch Halogen mit einer Atomnummer von bis und mit 35 substituiertes Phenyl oder Thienyl ist, sowie Salze solcher Verbindungen mit salzbildenden Eigenschaften.

12. Verbindungen der Formel I gemäss Anspruch 1, worin R für in 2-und/oder 3-Stellung durch Halogen mit einer Atomnummer von bis und mit 35 mono- oder disubstituiertes oder durch Nitro oder Cyan monosubstituiertes Phenyl steht, $R_1$, $R_2$ und $R_3$ Niederalkyl mit bis zu 4 Kohlenstoffatomen bedeuten, X für Sauerstoff steht, $Alk_1$ und $Alk_2$ für unsubstituiertes α,ω-Alkylen mit 2 oder 3 Kohlenstoffatomen stehen, $R_4$ für Niederalkyl mit bis zu 4 Kohlenstoffatomen steht, Z eine einfache Bindung ist, Y für die Gruppe -(C=O)- steht und $Ar_1$ durch Halogen mit einer Atomnummer von bis und mit 35 monosubstituiertes Phenyl ist, sowie Salze solcher Verbindungen mit salzbildenden Eigenschaften.

13. 2,6-Dimethyl-4-(3-nitrophenyl)-1,4-dihydropyridin-3,5-dicarbonsäure-3-methylester-5-[2-[N-(4-(4-fluorphenyl)-4-oxo-n-butyl)-N-methyl-amino]äthylester] oder dessen Salze.

14. 2,6-Dimethyl-4-(3-nitrophenyl)-1,4-dihydropyridin-3,5-dicarbonsäure-3-methylester-5-[2-[N-(4-(4-fluorphenyl)-1,4-dioxo-n-butyl)-N-methyl-amino]äthylester],
2,6-Dimethyl-4-(3-nitrophenyl)-1,4-dihydropyridin-3,5-dicarbonsäure-3-methylester-5-[2-[N-äthyl-N-(4-(4-fluorphenyl)-4-oxo-n-butyl)amino]äthylester],
2,6-Dimethyl-4-(3-nitrophenyl)-1,4-dihydropyridin-3,5-dicarbonsäure-3-methylester-5-[2-[N-benzyl-N-(4-(4-fluorphenyl)-4-oxo-n-butyl)amino]äthylester],
2,6-Dimethyl-4-(3-nitrophenyl)-1,4-dihydropyridin-3,5-dicarbonsäure-3-methylester-5-[2-[N-methyl-N-(4-phenyl-1,4-dioxo-n-butyl)amino]äthylester],
2,6-Dimethyl-4-(3-nitrophenyl)-1,4-dihydropyridin-3,5-dicarbonsäure-3-methylester-5-[2-[N-methyl-N-(4-phenyl-4-oxo-n-butyl)amino]-äthylester],
2,6-Dimethyl-4-(3-nitrophenyl)-1,4-dihydropyridin-3,5-dicarbonsäure-3-methylester-5-[2-[N-benzyl-N-(4-phenyl-4-oxo-n-butyl)amino]-äthylester],

2,6-Dimethyl-4-(3-nitrophenyl)-1,4-dihydropyridin-3,5-dicarbonsäure-3-methylester-5-[2-[N-methyl-N-(3-(2-thenoyl)propyl)amino]-äthylester],
2,6-Dimethyl-4-(3-nitrophenyl)-1,4-dihydropyridin-3,5-dicarbonsäure-3-methylester-5-[2-[N-benzyl-N-(3-(2-thenoyl)propyl)amino]-äthylester],
oder deren Salze.

15. 2,6-Dimethyl-4-(3-nitrophenyl)-1,4-dihydropyridin-3,5-dicarbonsäure-3-methylester-5-[2-[N-(4-(4-fluorphenyl)-4-oxo-n-butyl)-N-methyl-amino]-2-phenyl-äthylester], 2,6-Dimethyl-4-(3-nitrophenyl)-1,4-dihydropyridin-3,5-dicarbonsäure-3-methylester-5-[2-[N-(4-(4-fluorphenyl)-4-oxo-n-butyl)-N-methyl-amino]-1-phenyl-äthylester] oder deren Salze.

16. Pharmazeutisch verwendbare, nicht-toxische Säureadditionssalze von Verbindungen der Ansprüche 1 - 15.

17. Verbindungen gemäss den Ansprüchen 1 - 16 mit coronardilatatorischen und antihypertensiven Eigenschaften.

18. Verbindungen gemäss den Ansprüchen 1 - 16 zur Anwendung in einem Verfahren zur therapeutischen Behandlung des menschlichen oder tierischen Körpers.

19. Pharmazeutische Präparate enthaltend Verbindungen der Ansprüche 1 - 16.

20. Verwendung von Verbindungen gemäss den Ansprüchen 1 - 16 zur Herstellung von pharmazeutischen Präparaten.

21. Verwendung von Verbindungen gemäss den Ansprüchen 1 - 16 zur Herstellung von Arzneimitteln als Coronardilatatoren und Antihypertensiva zur Behandlung von cardiovasculären Krankheitszuständen, wie Angina pectoris und ihre Folgezustände, Gefäss-Spasmen,

zentrale und periphere Durchblutungsstörungen, hoher Blutdruck, Arrhythmien, Herzinsuffizienz sowie Migräne, ferner zur Anwendung als Hemmer der Plättchenaggregation.

22. Verfahren zur Herstellung von Verbindungen der Formel I gemäss Anspruch 1 und Salzen davon, dadurch gekennzeichnet, dass man

a) eine Verbindung der Formel

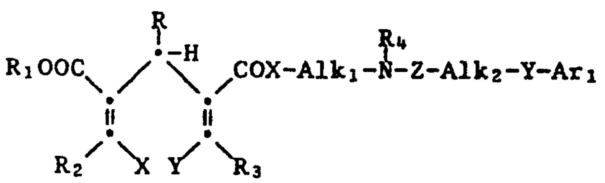

(II),

worin einer der Reste X und Y für die Gruppe der Formel $-NH_2$ steht und der andere Hydroxy oder die Gruppe der Formel $-NH_2$ bedeutet, oder ein Tautomeres davon oder ein entsprechendes Tautomerengemisch ringschliesst, oder

b) eine Verbindung der Formel R-CHO (III) oder ein reaktionsfähiges funktionelles Derivat davon mit einer Verbindung der Formel

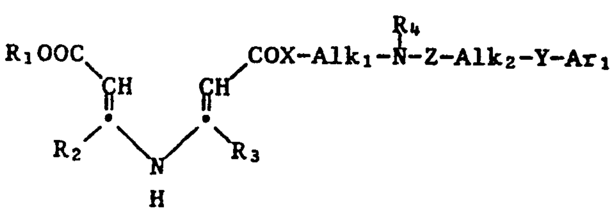

(IV)

oder einem Tautomeren davon oder einem entsprechenden Tautomerengemisch umsetzt, oder

c) in einer Verbindung der Formel

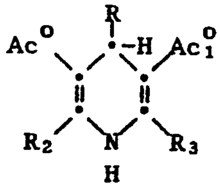

(V),

in welcher einer der Reste $Ac^{o}$ und $Ac_1^{o}$ eine in die Gruppe $-COOR_1$ bzw. in den Rest der Formel

$$-COX-Alk_1-\overset{R_4}{\overset{|}{N}}-Z-Alk_2-Y-Ar_1 \qquad (Va)$$

überführbare Gruppe bedeutet, und der andere die Gruppe $-COOR_1$ oder die Gruppe der Formel Va oder einen in die Gruppe $-COOR_1$ bzw. die Gruppe der Formel Va überführbaren Rest bedeutet, den Rest $Ac^{o}$ in die Gruppe $-COOR_1$ und/oder den Rest $Ac_1^{o}$ in einen Rest der Formel Va überführt, oder

d) eine Verbindung der Formel

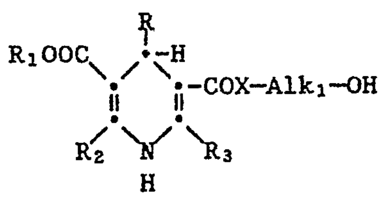

(VI)

oder einen reaktionsfähigen Ester davon, mit einer Verbindung der Formel

$$H\overset{R_4}{\overset{|}{N}}-Z-Alk_2-Y-Ar_1 \qquad (VIa)$$

umsetzt, oder

e) eine Verbindung der Formel

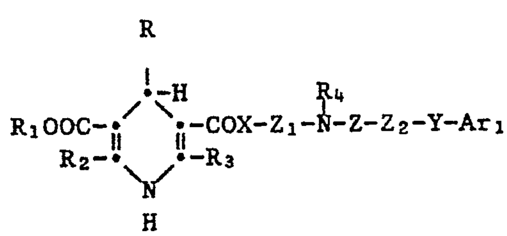

(VII)

worin mindestens eine der Gruppen $Z_1$ und $Z_2$ eine mittels Reduktion in die Gruppe $Alk_1$ bzw. $Alk_2$ überführbare Gruppe bedeutet und die andere die Gruppe $Alk_1$ bzw. $Alk_2$ oder eine mittels Reduktion in die

Gruppe $Alk_1$ bzw. $Alk_2$ überführbare Gruppe darstellt, die Gruppe $Z_1$ und/oder die Gruppe $Z_2$ mittels Reduktion in die Gruppe $Alk_1$ bzw. $Alk_2$ überführt, oder

f) eine Verbindung der Formel

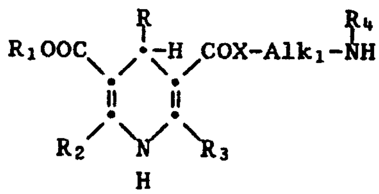

(VIII),

oder ein Salz davon, mit einer Verbindung der Formel

$$HO-Z-Alk_2-Y-Ar_1 \quad (IX),$$

oder einem reaktionsfähigen Derivat davon, umsetzt, oder

g) in einer Verbindung der Formel

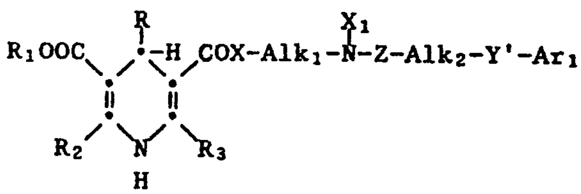

(X),

worin $X_1$ die Bedeutung von $R_4$ hat oder eine abspaltbare und durch Wasserstoff ersetzbare Gruppe bedeutet, Y' die Bedeutung von Y hat oder eine in die Gruppe Y überführbare Gruppe darstellt, wobei mindestens eine abspaltbare und durch Wasserstoff ersetzbare Gruppe $X_1$ abgespalten und durch Wasserstoff ersetzt und/oder eine von Y verschiedene Gruppe Y' in die Gruppe Y umgewandelt wird, die von $R_4$ verschiedene Gruppe $X_1$ abspaltet und durch Wasserstoff ersetzt, und/oder die von Y verschiedene Gruppe Y' in die Gruppe Y umwandelt, gegebenenfalls weitere an funktionelle Gruppen gebundene Schutzgruppen abspaltet und durch Wasserstoff ersetzt, wobei die Ausgangsstoffe der Formel II bis X, sofern sie salzbildende Eigenschaften aufweisen, auch in Form ihrer Salze verwendet werden können, und die Gruppen R, $R_1$, $R_2$, $R_3$, $R_4$, X, Z, $Alk_1$, $Alk_2$, Y und $Ar_1$ die unter der Formel I angegebenen Bedeutungen haben, und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden basischen Eigenschaften in ein Salz umwandelt, und/ oder, wenn erwünscht, ein erhaltenes Racematgemisch in die reinen Racemate bzw. Diastereomeren und/oder ein erhaltenes Racemat in die optischen Antipoden auftrennt.

23. Die nach dem Verfahren des Anspruchs 22 erhältlichen Verbindungen.

FO 7.4 JL/cs*

0214094

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung: EP 86810349.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A1 - 0 145 956 (CIBA-GEIGY) * Zusammenfassung; Ansprüche 1, 21,23,25 * -- | 1,17, 19,21, 22 | C 07 D 211/90<br>C 07 D 409/12<br>A 61 K 31/44 |
| A | EP - A2 - 0 151 006 (YAMANOUCHI) * Zusammenfassung; Ansprüche 1, 4,5,7 * ---- | 1,17, 19,21 | |

RECHERCHIERTE SACHGEBIETE (Int. Cl.4)

C 07 D 211/00
C 07 D 409/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-22
Unvollständig recherchierte Patentansprüche: —
Nicht recherchierte Patentansprüche: 23
Grund für die Beschränkung der Recherche:

Unklarheiten

| Recherchenort | Abschlußdatum der Recherche | Prüfer |
|---|---|---|
| WIEN | 18-11-1986 | HOCHHAUSER |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument